# EUROPEAN PATENT APPLICATION

(11) **EP 2 057 996 A2**
(43) Date of publication of application: **13.05.2009**
(21) Application number: 09075040.7
(22) Date of filing: 17.03.2005
(51) Int. Cl.: A61K 38/17, C07K 14/575, A61P 3/00

(54) **Y4 selective receptor agonists for therapeutic intervention**

(30) Priority: 17.03.2004 DK 200400436; 01.04.2004 US 558932 P; 06.07.2004 US 585964 P; 14.02.2005 GB 0503110
(62) Divisional of application: 05716258.8
(71) Applicant: 7TM Pharma A/S, 2970 Hoersholm (DK)
(72) Inventor: Schwartz, Thue, 2000 Frederiksberg (DK)
(74) Representative: Walls, Alan James

(57) **Abstract**

Y4 receptor agonists selective for the Y4 receptor over the Y1 and Y2 receptors are useful for treatment of conditions responsive to activation of Y4 receptors. The Y4 selective agonists (a) are PP-fold peptide or PP-fold peptide mimics which have C- and N-terminal sequence features as specified in the description or (b) have an covalent intramolecular link, or (c) comprise two covalently linked C-terminal Y4 receptor-recognition amino acid sequences each of which comprises the last four residues of a C-terminal receptor recognition sequence of the type (a) agonists.

## Description

### FIELD OF THE INVENTION

The invention relates to peptide or peptidic compounds that act as selective agonists of the Y4 relative to the Y1 and Y2 receptors, and to their use in treatment of conditions responsive to activation of Y4 receptors, for example for treatment of obesity and overweight, and conditions in which these are considered contributory factors, diarrhoea and intestinal hypersecretion.

### BACKGROUND TO THE INVENTION

**The PP-fold family of peptides -** NPY (Neuropeptide Y) (human sequence - SEQ ID. No:1), PYY (Peptide YY) (human sequence- SEQ ID. No:2), and PP (Pancreatic Polypeptide) (human sequence - SEQ ID. No:3), are naturally secreted homologous, 36 amino acid, C-terminally amidated peptides, which are characterized by a common three-dimensional, structure - the PP-fold - which is surprisingly stable even in dilute aqueous solution and is important for the receptor recognition of the peptides.
Initially the X-ray structure of avian PP was characterized in great detail through X-ray crystallographic analysis down to a resolution of 0.98 A and the unique structure obtained its name from this peptide (Blundell et al. 1981 Proc.Natl.Acad.Sci.USA 78: 4175-79*;* Glover et al. 1984, Eur.J.Biochem. 142: 379-85). Subsequently, the PP-fold structure of other members of the family have been analysed through especially NMR spectroscopic analysis. Both X-ray and NMR analysis are obviously performed in very concentrated or solid conditions; however, detailed circular dichroism analysis suggests that NPY and PP even in aqueous solution adopt the PP-fold structure, which is unusual for such a small peptide (Fuhlendorff et al. 1990 J.Biol.Chem. 265: 11706-12). Importantly, analysis of the proteolytic stability of the peptides and fragments and analogs of these strongly indicate that for example the full length PP1-36 even in dilute aqueous solution is held in a folded configuration which protects it from degradation by certain enzymes which readily and rapidly degrade analogs which cannot adopt the PP-fold structure due to minor substitutions (Schwartz et al. 1990 Annals NY Acad.Sci. 611: 35-47).

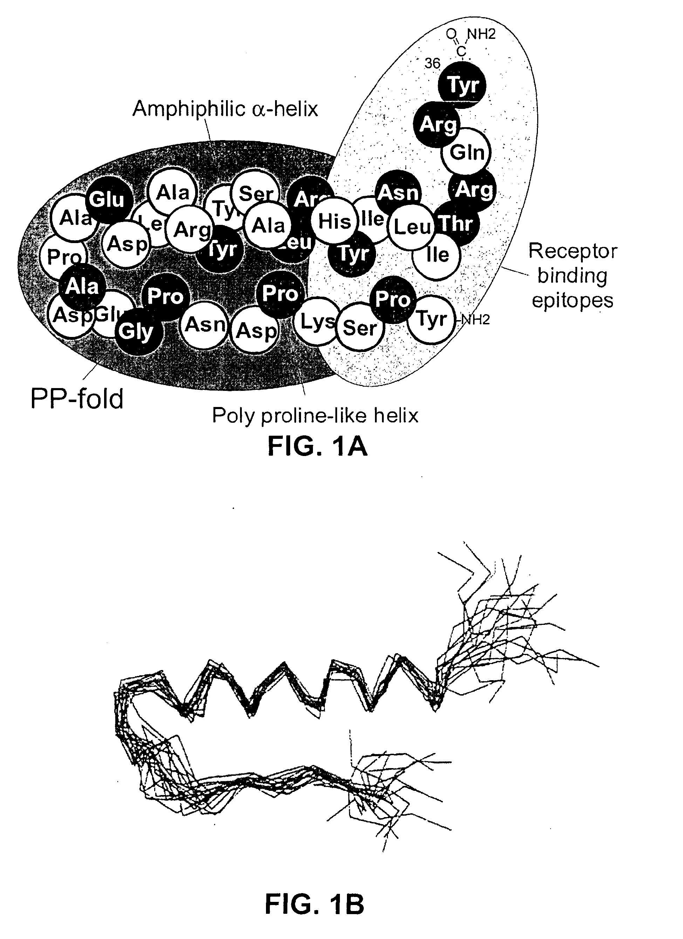

The PP-fold structure common to NPY, PYY and PP consists of 1) an N-terminal polyproline-like helix (corresponding to residues 1 through 8 with Pro2, Pro5, and Pro8) followed by 2) a type I beta-turn region (corresponding to residues 9 through 12) followed by 3) an amphiphilic alpha-helix (residues 13-30) which lies anti-parallel to the polyproline helix with an angle of about 152 degrees between the helical axes, and 4) a C-terminal hexapeptide (residues 31-36). The folded structure is stabilized through hydrophobic interactions between side chains of the amphiphilic alpha-helix which are closely interdigitating with the three hydrophobic proline residues (*Schwartz et al 1990*). Besides key residues in the receptor recognizing C-terminal hexapeptide it is the core hydrophobic residues, which stabilize the PP-fold structure, which are conserved across the family of PP-fold peptides. Fig. 1A depicts the NPY sequence, with residues which are conserved amongst NPY, PYY and PP shown as white text on dark background. Fig. 1A also illustrates the elements of the PP-fold structure described above. The C-terminal hexapeptide, which is important for receptor recognition is unstructured, but the PP fold provides a stable scaffold, which presents the C-terminal hexapeptide to the receptors (illustrated in Fig. 1 B), which to variable degree are dependent or independent also upon parts of the N-terminus of the peptides. NMR spectroscopic analysis has demonstrated that the far C- and the N-terminal parts of for example NPY are rather mobile, meaning that the PP-fold is constantly in danger of being "unzipped" from the free terminal end.

**NPY** is a very wide-spread neuropeptide with multiple actions in various parts of both the central and peripheral nervous system acting through a number of different receptor subtypes in man: Y1, Y2, Y4 and Y5. The main NPY receptors are the Y1 receptor, which generally is the post-synaptic receptor conveying the "action" of the NPY neurones and the Y2 receptor which generally is a pre-synaptic, inhibitory receptor. This is also the case in the hypothalamus, where NPY neurones - which also express the melanocortin receptor antagonist / inverse agonist AgRP (agouti related peptide) - act as the primary "sensory" neurones in the stimulatory branch of the arcuate nucleus. Thus, in this the "sensor nucleus" for the control of appetite and energy expenditure, the NPY/AgRP neurones together with the inhibitory POMC/CART neurones monitor the hormonal and nutritional status of the body as these neurones are the target for both the long-term regulators such as leptin and insulin and short term regulators such as ghrelin and PYY (see below). The stimulatory NPY/AgRP neurones project for example to the paraventricular nucleus - also of the hypothalamus - where its postsynaptic target receptors are believed to be Y1 and Y5 receptors. NPY is the most potent compound known in respect of increasing food intake, as rodents upon intracerebroventricular (ICV) injection of NPY will eat until they literally burst. AgRP from the NPY/AgRP neurones acts as an antagonist mainly on melanocortin receptors type 4 (MC-4) and block the action of POMC derived peptides - mainly aMSH - on this receptor. Since the MC4 receptor signal acts as an inhibitor of food intake, the action of AgRP is - just like the NPY action - a stimulatory signal for food intake (i.e. an inhibition of an inhibition). On the NPY/AGRP neurons are found inhibitory - pre-synaptic - Y2 receptors, which are the target both of locally released NPY as well as a target for the gut hormone PYY - another PP-fold peptide.

**PYY** is released during a meal - in proportion to the calorie content of the meal - from entero-endocrine cells in the distal small intestine and the colon, to act both in the periphery on GI-tract functions and centrally as a satiety signal. Peripherally, PYY is believed to function as an inhibitor - an "illeal break" - on for example upper GI-tract motility, gastric acid and exocrine pancreatic secretion. Centrally, PYY is believed to act mainly on the presynaptic, inhibitory Y2 receptors on the NPY/AgRP neurones in the arcuate nucleus, which it is believed get access to from the blood *(*Batterham et al. 2002 Nature 418: 650-4*).* The peptide is released as PYY1-36, but a fraction - approximately 50 % - circulates as PYY3-36 which is a product of degradation by dipeptidylpeptidase-IV an enzyme which removes a dipeptide from the N-terminus of a peptide provided that a Pro or Ala is found in position two as in all three PP-fold peptides - PP, PYY and NPY (Eberlein et al. 1989 Peptides 10: 797-803). Thus PYY in the circulation is a mixture of PYY1-36, which acts on both Y1 and Y2 receptors (as well as Y4 and Y5 with various affinities), and PYY3-36 - which has lower affinities for the Y1, Y4 and Y5 receptors than for the Y2 receptor.

**PP** is a hormone, which is released from endocrine cells in the pancreatic islets, almost exclusively governed by vagal cholinergic stimuli elicited by especially food intake (Schwartz 1983 Gastroenterology 85:1411-25). PP has various effects on the gastrointestinal tract, but none of these are observed in isolated cells and organs, and all appear to be dependent on an intact vagal nerve supply (Schwartz1983 Gastroenterology 85:1411-25). In accordance with this, the PP receptors, which are called Y4 receptors, are located in the brain stem with a strong expression in vagal motor neurones - activation of which results in the peripheral effects of PP - and in the nucleus tractus solitarirus (NTS) - activation of which results in the effects of PP as a satiety hormone (Whitecomb et al. 1990 Am.J.Physiol. 259: G687-91*,* Larsen & Kristensen 1997 Brain Res.Mol.Brain Res 48: 1-6). It should be noted that PP from the blood has access to this area of the brain since the blood brain barrier is "leaky" in this area where various hormones from the periphery are sensed. Recently it has been argued that part of the effect of PP on food intake is mediated through an action on neurones - especially the POMC/CART neurones in the arcuate nucleus (Batterham et al. 2004 Abstract 3.3 International NPY Symposium in Coimbra, Portugal). PP acts through Y4 receptors for which it has a subnanomolar affinity as opposed to PYY and NPY which have nanomolar affinity for this receptor (Michel et al. 1998 Pharmacol. Rev. 50: 143-150). PP also has an appreciable affinity for the Y5 receptor, but it is not likely of physiological importance in relation to circulating PP due to both lack of access to the cells in the CNS where this receptor especially is expressed and due to the relatively low affinity for PP.

### PP-fold peptide receptors

There are four well established types of PP-fold peptide receptors in man: Y1, Y2, Y4, and Y5 which all recognize NPY1-36 and PYY1-36 with similar affinity. At one time a Y3 receptor type, which might prefer NPY over PYY, was suggested, but today this is not accepted as a real receptor subtype (Michel et al. 1998 Pharmacol. Rev. 50: 143-150). A Y6 receptor subtype has been cloned, which in man is expressed in a truncated form lacking TM-VII as well as the receptor tail and consequently at least on its own does not appear to form a functional receptor molecule.

**Y1 receptors** - affinity studies suggest Y1 binds NPY and PYY equally well and basically not PP. Affinity for Y1 is dependent on the identities of both end sequences of the PP-fold molecule (NPY/PYY) - for example residues Tyr1 and Pro2 are essential - and it is dependent on the peptide ends being presented in just the right way. In the C-terminal end, where the side-chains of several of the residues are essential, the Y1 receptor - like the Y5 and Y4 receptor but not the Y2 receptor - tolerates certain substitutions in position 34 (normally a Gln) - such as Pro (Fuhlendorff et al. 1990 J.Biol.Chem. 265: 11706-12*,* Schwartz et al. 1990 Annals NY Acad.Sci. 61: 35-47). Some structure-function studies concerning the requirements of the Y1 and Y2 receptors have been reported (Beck-Sickinger et al. 1994 Eur.J.Biochem. 225: 947-58*;* Beck-Sickinger and Jung 1995 Biopolymers 37: 123-42*;* Söll et al. 2001 Eur.J.Biochem. 268: 2828-37).

**Y2 receptors** - affinity studies suggest Y2 binds NPY and PYY equally well and basically not PP. The receptor requires especially the C-terminal end of the PP-fold peptide (NPY/PYY). Thus, long C-terminal fragments - down to for example NPY13-36 (the whole alpha helix plus the C-terminal hexapeptide) - are recognized with relatively high affinity, i.e. to within ten-fold of the affinity of the full-length peptide (Sheikh et al. 1989 FEBS Lett. 245: 209-14*,* Sheikh et al. 1989 J.Biol.Chem. 264: 6648-54)*.* Therefore various N-terminal deletions, which eliminate the binding to the Y1 receptor, still preserve some degree of binding to the Y2 receptor. However, the affinity of the C-terminal fragments is reduced approximately 10 fold as compared to NPY / PYY for even relatively long fragments. The Gln residue in position 34 of NPY and PYY is highly important for the ligand recognition of the Y2 receptor (Schwartz et al. 1990 Annals NY Acad.Sci. 611: 35-4 7).

**Y4 receptors** - affinity studies suggest that Y4 binds PP with subnanomolar affinity corresponding to the concentrations found in plasma whereas NPY and PYY are recognized with much lower affinity. Such studies suggest the Y4 receptor is highly dependent on the C-terminal end of the PP-fold peptides, and that relatively short N-terminal deletions impairs the affinities of the ligands. Some structure activity studies concerning the Y4 receptor have been reported (Gehlert et al. 1996 Mol.Pharmacol.50: 112-18*;* Walker et al. 1997 Peptides 18: 609-12).

**Y5 receptors** - affinity studies suggest that Y5 binds NPY and PYY equally well, and also binds PP with lower affinity, which however is below the normal circulating levels of this hormone. PYY3-36 is also recognized well by the Y5 receptor, however this receptor is to a large degree expressed in the CNS where such peptide cannot get access to the receptor readily when administered in the periphery.

PP-fold peptides and analogs of these have been suggested for use in the treatment of obesity and associated diseases, including for example Prader Willi's syndrome, based on the demonstrated effects of certain of the these peptides in animal models and in man and on the fact that obese people have low basal levels of PP and PYY as well as lower meal responses of these peptides (Holst JJ et al. 1983 Int.J.Obes. 7: 529-38*;* Batterham et al. 1990 Nature). It has been known since the mid seventies that PP could affect food intake in rodents. In 1993 it was reported that infusion of PP in morbidly obese patients with Prader Willi's syndrome decreased food intake (Berntson et al. 1993 Peptides 14: 497-503). Recently this effect of PP was confirmed by infusion of PP in normal human subjects where a long lasting suppression of appetite and reduced food intake over 24 hours was observed *(*Batterham et al 2003, Clin.Endocrinol.Metab. 88: 3989-92).

However, the native PP-fold peptides are not optimal for use as biopharmaceuticals, for example they are susceptible to degradation by various proteases such as DPP-IV. Thus, the natural peptides are not optimized for protein stability as they are made to normally act for a relatively short time as a neuropeptide or hormone.

For the treatment of conditions responsive to Y4 receptor modulation, such as obesity and intestinal hyper-secretion it would therefore be desirable to use PP-fold peptides or PP-fold peptide mimics, which acted as agonists and were specific for the Y4 receptor intended as target, and which stably preserve elements of the PP-fold structure important for receptor binding. In particular, it would be highly desirable to use such agents which are selective for the Y4 receptor over the Y1 and Y2 receptors. This is particularly important, since activation of the Y1 receptor is expected to potentially cause unwanted cardiovascular and renal side effects such as vasoconstriction and natriuresis Moreover, activation of the Y2 receptor may also cause side effects. Although it is still unclear what the really efficient angiogenic Y receptor profile is, Y2 agonists such as NPY3-36 apparently can induce revascularization in for example ischemic hind limb models, i.e. when administered in high doses with constant exposure as for example released from inoperated pellets (Zukowska Z et al. Trends Cardiovasc Med. 2003 ,13 :86-92). The angiogenic response to NPY is reduced in Y2 receptor knock out animals; however, the response to this broad-spectrum Y receptor agonist NPY is in fact not eliminated and both Y2 and Y5 receptors are up-regulated in ischemic vessels (Lee et al J.Clin.Invest. 2003, 111: 1853-62). Nevertheless, a PP-fold peptide or PP-fold peptide mimic could through activation of the Y2 receptor cause side effects such as worsen the retinopathy for example in diabetic patients and could potentially aid in the neovascularization associated with the growth of certain cancers. Thus use of efficacious and selective Y4 receptor over Y1 and Y2 receptor agonists would be particularly useful in diseases and conditions susceptible to Y4 receptor activation.

### Some Common Terms Used In this Specification

**Affinity:** The affinity of a peptide to a specific receptor is given for example as an IC₅₀ value or a Kᵢ or K_{d} value, which in a specific, non-limiting example is determined in an assay, such as a competition binding assay. The IC50 value corresponds to the concentration of the peptide which displaces a - for the given receptor relevant - radioactive ligand used in an amount far less than the Kd for that radioactive ligand to 50%.

**Appetite:** A natural desire, or longing for food. Increased appetite generally leads to increased feeding behavior.

### Appetite Suppressants: Compounds that decrease the desire for food

**Binding:** A specific interaction between two molecules, such that the two molecules interact. Binding to a receptor can be specific and selective, so that one molecule is bound preferentially when compared to another molecule. Specific binding may be identified by a disassociation constant (K_{d}). This value is dependent on the selectivity of the compound tested. For example, a compound with a K_{d} that is less than 10 nM is generally considered an excellent drug candidate. However, a compound that has a lower affinity, but is selective for the particular receptor, can also be a good drug candidate.

**Body Mass Index (BMI):** A mathematical formula for measuring body mass, also sometimes called Quetelet's Index. BMI is calculated by dividing weight (in kg) by height² (in meters). The current standards for both men and women accepted as "normal" are a BMI of about 20 kg/m². In one embodiment, a BMI of greater than 25 kg/m² can be used to identify an obese subject. Grade I obesity corresponds to a BMI of 25 kg/m². Grade II obesity corresponds to a BMI of 30-40 kg/m²; and Grade III obesity corresponds to a BMI greater than 40 kg/m² (Jequier 1987 Ain. J Clin. Nutr. 45:1035-47). Ideal body weight will vary among species and individuals based on height, body build, bone structure, and sex.

**Caloric intake or calorie intake:** The number of calories (energy) consumed by an individual. In the present context this term is identical to the term "energy intake".

Cosmetic treatment: The term is intended to denote a treatment that is not for medical purposes, but for improving the well-being of a subject e.g. with respect to the appearance of a subject. Included in the term is treatment of a subject who desires to decrease his weight without necessarily being overweight or obese.

**Food intake:** The amount of food consumed by an individual. Food intake can be measured by volume or by weight. Included within its meaning is i) food intake as being the total amount of food consumed by an individual, and ii) food intake is the amount of proteins, fat, carbohydrates, cholesterol, vitamins, minerals, or any other food component, of the individual. Accordingly, the term food intake as used in the present context is similar to the term "energy intake".

**Normal Daily Diet:** The average food intake for an individual of a given species. A normal daily diet can be expressed in terms of caloric intake, protein intake, carbohydrate intake, and/or fat intake. A normal daily diet in humans generally comprises the following: about 2,000, about 2,400, or about 2,800 to significantly more calories. In addition, a normal daily diet in humans generally includes about 12 g to about 45 g of protein, about 120 g to about 610 g of carbohydrate, and about 11 g to about 90 g of fat. A low calorie diet would be no more than about 85%, and preferably no more than about 70%, of the normal caloric intake of a human individual. In animals, the caloric and nutrient requirements vary depending on the species and size of the animal. For example, in cats, the total caloric intake per kg, as well as the percent distribution of protein, carbohydrate and fat varies with the age of the cat and the reproductive state.

**Obesity:** A condition in which excess body fat may put a person at health risk (see Barlow and Dietz, Pediatrics 102:E29, 1998*; National Institutes of Health, National Heart, Lung, and Blood Institute (NHLBI),* Obes. Res. 6 (suppl. 2):51 S209S, 1998)*.*
Excess body fat is a result of an imbalance of energy intake and energy expenditure. In one embodiment, the Body Mass Index (BMI) is used to assess obesity. In one embodiment, a BMI of from about 22 kg/m² (i.e. about 10% above the normal value) and up to about 30 kg/m² is regarded as overweight, especially from about 25.0 kg/m² and up to 30 kg/m², while a BMI of 30 kg/m² or more is obese.

**Overweight:** An individual who weighs more than their ideal body weight. An overweight individual can be obese, but is not necessarily obese. In one embodiment, an overweight individual is any individual who desires to decrease their weight. In another embodiment, overweight individual is an individual with a BMI of a BMI of from about 22 kg/m² (i.e. about 10% above the normal value) and up to about 30 kg/m² is regarded as overweight, especially from about 25.0 kg/m² and up to 30 kg/m². It should be noted that subjects having a BMI that is only slightly higher than that of the normal value (e.g. from about 22 to about 25 kg/m²) very often have a desire to loose weight although this may only be for cosmetic reasons.

**Potency:** In vitro potency of a compound is defined in terms of EC₅₀ values, i.e. the concentration that leads to 50% of the maximally achievable effect as determined in a for the given receptor relevant signaling assay.

**Subject:** The subject can be any subject, including both human and veterinary mammalian subjects. Thus, the subject can be a human, or can be a non-human primate, a farm animal such as swine, cattle, sheep and poultry, a sport animal or pet such as dogs, cats, horses, hamsters, and rodents.

**Therapeutically effective amount:** A dose sufficient to prevent, treat or ameliorate a specific condition or disease and/or to alleviate specific signs or symptoms of a specific condition or disease. The term includes a dose that is sufficient or prevent advancement, or to cause regression of a disorder, or which is capable of relieving a sign or symptom of a disorder, or which is capable of achieving a desired result. In embodiments relating to cosmetic treatment or to treatment of overweight or obesity, a therapeutically effect of a receptor agonist is an amount sufficient to inhibit or halt weight gain, or an amount sufficient to decrease appetite, or an amount sufficient to reduce energy or food intake or increase energy expenditure. The term "cosmetically effective amount" is intended to denote a dose that is sufficient for the subject being treated to achieve a desired effect.

### DETAILED DESCRIPTION OF THE INVENTION

In its broadest aspect, the present invention provides the use of a Y4 receptor agonist other than PP, which is selective for the Y4 receptor over the Y1 and Y2 receptors, in the preparation of a composition for treatment of conditions responsive to activation of Y4 receptors
(a) the said agonist being a PP-fold peptide or PP-fold peptide mimic which has
   (i) a C-terminal Y4 receptor-recognition amino acid sequence represented by -X-Thr-Arg-X³-Arg-Tyr-C(=O)NR¹R² wherein R¹ and R¹ are independently hydrogen or C₁-C₆ alkyl X is Val, Ile, Leu or Ala, and X³ is a residue other than Gln, or a conservatively substituted variant thereof in which Thr is replaced by His or Asn and/or Tyr is replaced by Trp or Phe; and/or Arg is replaced by Lys, and
   (ii) an N-terminal Y receptor-recognition amino acid sequence represented by H₂N-X¹-Pro-X²-(Glu or Asp)- wherein X¹ is not present or is any amino acid residue, and X² is Leu, Ile or Ser or a conservative substitution thereof, or
(b) the said agonist comprising
   a C-terminal Y4 receptor-recognition sequence as defined in (i) above,
   said sequence being fused to an amphiphilic amino acid sequence domain comprising at least one alpha helical turn adjacent the N-terminus of the said hexapeptide sequence,
   said turn being constrained in a helical configuration by a covalent intramolecular link, and optionally
   an N- terminal sequence which commences with a Y4 receptor-recognition amino acid sequence as defined in (ii) above; or
(c) the said agonist comprising two covalently linked C-terminal Y4 receptor-recognition amino acid sequences each of which comprises the last four residues of the sequence defined in (i) above.

### Specific Invention-Related Terminology

The agonists with which this invention are concerned are **selective for the Y4 receptor over the Y1 and Y2 receptors.** In the present context, this condition is fulfilled if the agonist has an IC50 value that is at least 10-fold lower for the Y4 than for the Y1 and Y2 receptors when measured in the affinity assay described herein. In general, with respect to potency, the agonists of the invention also have EC50 values at least 10-fold lower for the Y4 than for the Y1 and Y2 receptors when measured in the potency assay described herein. Many of the preferred agonists of the invention have affinities and potencies at least 100-fold higher for the Y4 receptor than for the Y1 and Y2 receptors. Some of the preferred agonists of the invention have affinities and potencies at least 1000-fold higher for the Y4 receptor than for the Y1 and Y2 receptors.

For the purpose of this specification a **PP-fold peptide** is a molecule having a 3-D structure which, when mapped onto the original 3-D structure of avian PP as determined by X-ray crystallography (Blundell et al. 1981 Proc.Natl.Acad.Sci.USA 78: 4175-79*;* Glover et al. 1984, Eur.J.Biochem. 142: 379-85), has domains corresponding to, and substantially aligned as in, the N-terminal polyproline-like helix, the type I beta-turn region, the amphiphilic alpha-helix and the C-terminal hexapeptide domains of the said NPY, PYY and/or PP *(**Fig. 1**)*. The description of the different domains of the PP-fold peptides as used here thus refers to the original X-ray structure of avian PP (Blundell et al. 1981 Proc.Natl.Acad.Sci.USA 78: 4175-79*;* Glover et al. 1984, Eur.J.Biochem. 142: 379-85*; Schwartz et al 1990*).

For the purpose of this specification a **PP-fold peptide** mimic is a molecule having a 3-D structure which, when mapped onto the original 3-D structure of avian PP, at least has domains corresponding to, and substantially aligned as in, the last turn of the amphiphilic alpha-helix, and the C-terminal hexapeptide domains of PP. PP-fold peptide mimics may also, when mapped as aforesaid, have domains corresponding to one or more of the remaining turns of the amphiphilic alpha helix, the N-terminal polyproline-like helix, and the type I beta-turn region. A peptide mimic need not consist entirely of a sequence of alpha amino acids linked by classical peptide bonds. One or more of the bonds in such a sequence may be replaced by peptidomimetic bonds such as reverse amide, and reduced peptide bonds, so that the peptide mimetic may be regarded as a pseudopeptide sequence. Such bond replacements are capable of conferring resistance to endopeptidase degradation and improving pharmacodynamic properties of the molecule.
Such comparisons of 3-D structure as referred to in the above definitions of "PP-fold peptide" and "PP-fold peptide mimic", may be made by constructing models of the comparison molecules based on their atomic coordinates as determined by for example X-ray diffraction methods, or by use of one or more of the computer programs, which are commercially available for visualization of the predicted 3-D structure of a molecule from its structural formula, for example: "Maestro Modelling Environment" from Schrödinger Inc, 1500 S.W. First Avenue, Suite 1180 Portland, OR 97201; "Insight II Modeling Environment", Release 4.0, from Accelrys Inc. San Diego; and "SYBYL® 7.0" from Tripos Inc., 1699 South Hanley Rd., St. Louis, Missouri, 63144, USA. It should be noted that the 3-D structure of a PP-fold peptide or PP-fold peptide mimic according to the present invention need not, and generally will not, have an exact correspondence with that of a natural NPY, PPY, or PP. The apparent 3-D structure of a PP-fold peptide or PP-fold peptide mimic will vary depending on the experimental conditions used to study this and especially for smaller peptides may appear more or less unfolded under certain conditions. However, it is sufficient that the PP-fold peptide or PP-fold peptide mimic should have domains corresponding to those PP-fold domains specified above, and that it has the structural elements to adopt an overall shape similar to the native peptide in which the C-terminal and if present N-terminal sequences are normally orientated.

The agonists with which the invention is concerned have a **C-terminal Y4 receptor-recognition sequence.** This is a sequence, usually about 5-7 residues long and especially a hexapaptide sequence, located at the C-terminus of the agonist, which when present in a PP-fold peptide or PP-fold peptide mimic, binds to the Y4 receptor and activates the receptor through that binding interaction alone, or as a result of that binding action and the binding to the Y4 receptor of an N-terminal Y4 receptor sequence present in the agonist. An **N-terminal Y4 receptor-recognition sequence** is a sequence, usually about 3-5 residues long and especially a 4-residue sequence, located at the N-terminus of the agonist, which when present in a PP-fold peptide or PP-fold peptide mimic, binds to the Y4 receptor and activates the receptor through a combination of that binding action and the binding to the Y4 receptor of a C-terminal Y4 receptor sequence present in the agonist. The classic C- and N-terminal Y4 receptor-recognition sequences are those found in the natural PP peptide, but as will become apparent herein, these classic sequences may be modified to retain Y4 recognition but reduce Y1 and Y2 recognition. A C- or N-terminal sequence present in any particular agonist is a Y4 receptor-recognition sequence if the PP-fold peptide or PP-fold peptide mimic in question interacts with the Y4 receptor in the affinity and/or potency assays described herein, when that sequence is present but not (or to no significant extent) when that sequence is deleted.

In this specification, terminology such as "residue corresponding to Ser3 of NPY" is a reference to the amino acid residue of the agonist which, on mapping the 3-D structure of the agonist onto that of NPY, most closely maps to Ser3 of NPY. Similarly, terminology such as "in a position corresponding to Pro34 of PP" is a reference to the position in the agonist of the amino acid which, on mapping the 3-D structure of the agonist onto that of PP, most closely maps to Pro34 of PP. The actual numbering of a particular residue in a specific peptide may vary from this due to the occurrence of for example deletions in respect of the natural peptides.

In general, PP-fold or PP-fold mimic agonists with which the invention is concerned will usually have a peptidic backbone or a backbone which is partly peptidic, at least in having a C-terminal amino acid sequence and often an N-terminal amino acid sequence, although the remainder of the backbone may be a non-peptidic linker radical, for example a straight or branched alkylene chain. Amino acids present in the peptidic portion(s) of the agonists will usually be naturally occurring, especially in C-and N-terminal sequences which interact with the Y4 receptor, but non-natural alpha amino acids which preserve the PP-fold and do not prevent Y4 receptor binding may also be present.

When the agonists with which the invention is concerned have C- or N- terminal amino acid sequences, the C-termini may be amidated and/or the N-termini may be acylated, to confer resistance to carboxy- and/or aminopeptidases. In fact, the C-terminus of the native NPY, PYY and PP peptides are amidated, so a C-terminal amino acid of an agonist of the invention may also be amidated.

In this specification, reference is made to amino acids by their common names or abbreviations, such as valine (Val), leucine (Leu), isoleucine (Ile), methionine (Met), phenylalanine (Phe), asparagine (Asn), glutamic acid (Glu), glutamine (Gln), histidine (His), lysine (Lys), arginine (Arg), aspartic acid (Asp), glycine (Gly), alanine (Ala), serine (Ser), threonine (Thr), tyrosine (Tyr), tryptophane (Trp), cysteine (Cys) and proline (Pro). When referred to by its common name or abbreviation, without specifying its steroisomeric form, the amino acid in question is to be understood as the L-form. Where the D-form is intended, the amino acid will be specifically referred to as such. Occasionally, where the context makes it desirable to do so, the L-form will be specified rather than inferred.

The term "conservative substitution" as used herein denotes that one or more amino acids is replaced by another, biologically similar residue. Examples include substitution of amino acid residues with similar characteristics, e.g. small amino acids, acidic amino acids, polar amino acids, basic amino acids, hydrophobic amino acids and aromatic amino acids. Non-limiting examples of conservative amino acid substitutions suitable for use in the present invention include those in the following Table and analogous substitutions of the original residue by non-natural alpha amino acids which have similar characteristics. For example, in a preferred embodiment of the invention Met residues are substituted with norleucine (Nle) which is a bioisostere for Met, but which - as opposed to Met - is not readily oxidised. Another example of a conservative substitution with a residue normally not found in endogenous, mammalian peptides and proteins would be the conservative substitution of Arg or Lys with for example, ornithine, canavanine, aminoethylcysteine or other basic amino acid. For further information concerning phenotypically silent substitutions in peptides and proteins, see, for example, Bowie et.al. Science 247, 1306-1310, 1990*.*

| Original residue | Conservative substitution |
|---|---|
| Ala | Gly |
| Arg | Lys |
| Asn | Gln, His, Thr |
| Asp | Glu |
| Gln | Asn, His |
| Glu | Asp |
| His | Asn, Gln |
| Ile | Leu, Val |
| Leu | Ile, Val |
| Lys | Arg |
| Met | Leu, Ile |
| Phe | Tyr, Trp, His |
| Ser | Thr, Asn |
| Thr | Ser, Asn, Gln |
| Trp | Tyr, Phe, His |
| Tyr | Trp, Phe, His |
| Val | Ile, Leu |

Unless otherwise specified in the context in which it occurs, the term "substituted" as applied to any moiety herein means substituted with up to four compatible substituents, each of which independently may be, for example, (C₁-C₆)alkyl, (C₁-C₆)alkoxy, hydroxy, hydroxy(C₁-C₆)alkyl, mercapto, mercapto(C₁-C₆)alkyl, (C₁-C₆)alkylthio, halo (including fluoro, bromo and chloro), trifluoromethyl, trifluoromethoxy, nitro, nitrile (-CN), oxo, phenyl, -COOH, -COOR^{A}, -COR^{A}, -SO₂R^{A}, -CONH₂, -SO₂NH₂, -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A}, -NHCOOR^{A}, -NR^{B}COOR^{A}, -NHSO₂OR^{A}, -NR^{B}SO₂OH, -NR^{B}SO₂OR^{A},-NHCONH₂, -NR^{A}CONH₂, -NHCONHR^{B}_{,} -NR^{A}CONHR^{B}, -NHCONR^{A}R^{B}, or -NR^{A}CONR^{A}R^{B} wherein R^{A} and R^{B} are independently a (C₁-C₆)alkyl group. An "optional substituent" may be one of the foregoing substituent groups.

Unless the context dictates otherwise, references herein to NPY, PYY and PP peptides and their sequences relate to the human forms of those peptides and their sequences. However, other mammalian NPY, PYY and PP peptides will often constitute PP-fold peptide mimics of human NPY, PYY and PP, or conservatively substituted human NPY, PYY or PP, as those terms are used herein.

Agonists with which the invention is concerned have a residue other than Gln, and preferably other than Asn, in a position corresponding to Pro34 of PP in their C-terminal Y4 receptor-recognition sequence. Avoidance of these residues helps to ensure reduced affinity for the Y2 receptors.

Furthermore an N-terminal Y receptor-recognition amino acid sequence when present in the agonists with which the invention is concerned is represented by H₂N-X¹-Pro-X²-(Glu or Asp)- wherein X¹ is not present or is any amino acid residue, and X² is Leu, Ile or Ser or a conservative substitution thereof. These requirements helps to ensure affinity of the N-terminal Y4 recognition sequence and to obtain selectivity against the Y1 and Y2 receptors.

### Type (a) agonists for use in accordance with the invention

In general, type (a) agonists are PP-fold analogues of PP, NPY or PYY, or PP-fold mimics of PP, NPY or PYY, which have modifications to retain or confer Y4 receptor potency, but to reduce substantially their potencies towards Y1 and Y2 receptors. Such PP-fold analogues and mimics include peptides having the full complement of PP-fold characteristics (N-terminal polyproline-like helix, beta turn, amphiphilic alpha helix and C-terminal hexapeptide), peptide analogues in which part of the backbone (for example the beta turn residues and adjacent residues) is replaced by a non peptidic spacer chain, and truncated peptides possessing the C-terminal hexapeptide and last turn of the amphiphilic alpha helix, but lacking all or part of the polyproline-like helix and/or beta turn.

***Type (b) agonists for use in accordance with the invention*** In general, type (b) agonists can be regarded as type (a) PP-fold peptide analogues or PP-fold mimics whose minimum PP-fold structural characteristics (the C-terminal hexapeptide and last turn of the alpha helix) are stabilised by a specified intramolecular covalent link.

Agonists of this type are characterised by an intramolecular link, which either has no equivalent in the native NPY, PYY or PP peptides or which correspond to or substitute a non-covalent interaction with a covalent link, for example the covalent, disulfide bridge between Cys2 and D-Cys27 in [Cys2,DCys27]-PP SEQ ID. No:4) mimics the non-covalent hydrophobic interaction between the side chains of Pro2 and Tyr27 as observed in the X-ray structure of avian PP. As stated, such a link can serve to stabilise essential elements of the PP-fold structure, particularly the mobile C-terminal Y4 recognition sequence and last turn of the alpha helix, and/or the presentation of the N-terminus. In the full length or close to full length peptides, such a link can stabilise the native PP-fold structure as such. This is beneficial in two ways, first it stabilizes the C-terminal part of the amphiphilic alpha-helix and thereby the presentation of the C-terminal Y4 recognition amino acid sequence in an optimal fashion resulting in high potency towards the Y4 receptor; secondly, by stabilizing the whole PP-fold as such the peptide becomes less susceptible to proteolytic degradation since such enzymes often require their target sequences to be found in a rather unfolded fashion (*Schwartz et al. 1990*). The intra-molecular link can also enable Y4-selective agonists which are heavily modified relative to the structure of the native peptides, for example agonists which lack one or more domains, or parts of domains, found in the native peptides.

One set of agonists of this type (b) has a PP-fold structure in which the helical turn-constraining intramolecular link extends from an amino acid residue in the amphiphilic domain to a linkage point in the N-terminal part of the agonist corresponding to the polyproline domain of a PP-fold peptide which extends antiparallel to the amphiphilic domain. The helical turn-constraining intramolecular link, for example a disulfide or lactam link, may extend from an amino acid residue in the amphiphilic domain to one of the four N-terminal residues.

An especially preferred subset of agonists of this type consists of PP-fold peptides with a helical turn-constraining intramolecular link extending from an amino acid residue in the amphiphilic domain to a linkage point in the N-terminal part of the polyproline domain. Such a link may be between residues in positions 5 and 20 or 8 and 16 or especially positions 2 and 27, for example a disulfide link between a D-Cys in position 27 and a Cys in position 2. The D-form of Cys is preferred in position 27 since this will orient the thiol side chain optimally to form a disulfide bridge with a "normal" L-from of Cys introduced in position 2 in order for the whole molecule to adopt and mimic the PP-fold structure. In another set of agonists of type (b), the helical turn-constraining intramolecular link extends between residues in the last helical turn of the alpha helix domain, for example a lactam link formed between Lys and Glu residues in the helical turn, or between a residue in the C-terminal Y4 recognition amino acid sequence and a residue in the last helical turn of the alpha helix domain, for example a lactam bridge between Lys and Glu in [Lys28,Glu32]PP25-36 (SEQ !D. No:5) or [Glu28,Lys32]PP25-36 (SEQ ID. No:6).

### Type (c) agonists for use in accordance with the invention

Type (c) agonists with which the invention is concerned may be regarded as dimers of at least the last 4 residues of the C-terminal Y4-receptor-recognition sequences present in type (a) and (b) agonists, for example dimers of the terminal hexapeptide sequence or for the last 5 or 4 residues. Thus, although the C-terminal Y4 receptor recognition amino acid sequence in itself - for example in the hexapeptide form - has rather low affinity for the Y4 receptor and in type (a) and (b) agonists has other structural elements to become a high affinity and potency ligand, it is possible to obtain useful high affinity and potency by joining two such C-terminal Y4 receptor recognition amino acid sequences in a dimer. Conceivably, the reason for the surprisingly high affinity of such constructs is that the Y4 receptor as many if not all other 7TM receptors of the family A type are found as and function as dimers at the cell membrane (Bouvier, 2001, Nat Rev Neurosci. 2: 274-86). Thus, although the affinity of a single C-terminal Y4 receptor recognition amino acid sequence is rather low for the Y4 receptor as such, the binding of one protomer of a dimeric form of such sequences will result in a "very high local concentration" of the other C-terminal Y4 receptor recognition amino acid sequence which will result in a high affinity of the dimeric ligand for the dimeric receptor. In the literature dimeric constructs of analogs of the C-terminal part of NPY have been made as antagonists for the Y1 receptor (Daniels et al. 1995 Proc Natl Acad Sci U S A. 92:9067-71). Surprisingly such constructs were subsequently shown to be agonists for the Y4 receptor (Parker et al. 1998 Eur J Pharmacol. 349:97-105).

The two sequences of the type (c) agonists may be linked, for example, by a crosslink between at least one pair of residues located at least 4 residues from the C terminus of each of the two sequences. For example, the two covalently linked sequences may comprise the last 5 or the last 6 residues of the C-terminal sequence defined and discussed above. In one embodiment, crosslinking is via a pair of crosslinks between two pairs of residues located at least 4 (for example 5 or 6) residues from the C terminus of each of the two sequences. Crosslinking methods include a disufide bridge between cysteine residues, or by an amide bond between the 3-amino group of a 2.3-propionic acid residue in one sequence and a carboxyl group of a side chain of a residue in the other sequence, or by a -(CH₂)₁₋₆- bridge formed by a bis-amino acid HOOCCH(NH₂)(CH₂)₁₋₆CH(NH₂)COOH, the ends of which form a residue in each of the respective sequences

An example of a type (c) agonist crosslinked by a single disulfide bridge is:

S-Cys-Thr-Arg-Pro-Arg-Tyr-CONH₂
|
S-Cys-Thr-Arg-Pro-Arg-Tyr-CONH₂ (SEQ ID. No:7)

However, the two C-terminal Y4 receptor recognition amino acid sequences of the dimer do not have to be identical. Thus another example of a type (c) agonist crosslinked by a single disulfide bridge is:

S-Cys-Thr-Arg-Pro-Arg-Tyr-CONH2
|
S-Cys-Thr-Arg-His-Arg-Tyr-CONH2 (SEQ ID. No:8)

Another example of a type (c) Y4 agonist hetero-dimer is:

S-Cys-[N-(N'-hexadecanoyl)-gammaglutamoyl]-Lys-Thr-Arg-Leu-Arg-Tyr-CONH2
|
S-Cys-Leu-Thr-Arg-Leu-Arg-Tyr-CONH2 (SEQ ID. No: 29).

In this peptide, a Lys residue has been introduced at the "31" position in one of the peptide units as an attachment side for - in this case - a serum albumin binding motif.

### C-terminal Y4 receptor-recognition sequences (Type (a), (b) and (c) agonists)

Being Y4 selective, all three types of agonist with which the invention is concerned have a C-terminal Y4 receptor-recognition amino acid sequence, which can be confirmed by the affinity and potency assays described herein.. One set of preferred C-terminal Y4 receptor-recognition amino acid sequences present in Type (a), (b) or (c) agonists of the invention is represented by -X-Thr-Arg-X³-Arg-Tyr-C(=O)NR¹R² where X and X³ are as previously defined and R¹ and R² are each hydrogen. Preferably also, residue X³ should not be Asn, and Lys, Arg, Asp or Glu are preferably avoided as X³ residues. Currently it is preferred that X³ is Pro, but X³ may also be His, or a non-natural Pro analogue selected from 4-hydroxyproline, azetidine-2-carboxylic acid, azetidine-3-carboxylic acid, azaproline, and 1-aminocyclobutanecarboxylic acid. In the C-terminal Y4 receptor-recognition amino acid sequence of the agonist, one preferred residue X is Leu.

In the agonists with which the invention is concerned, the C-terminal Y4 recognition sequence of 6 residues as defined and discussed above may be final residues of a C-terminal heptapeptide represented by -X^{A}-X-Thr-Arg-X³-Arg-Tyr-C(=O)NR¹R² wherein the residue X^{A} is non-basic and non-acidic, and the sequence -X-Thr-Arg-X³-Arg-Tyr-C(=O)NR¹R² is as defined and discussed above. In such cases, the said non-basic and non-acidic amino acid residue X^{A} may be, for example, Leu or Met.

Furthermore, the C-terminal heptapeptide sequence of the preceding paragraph may itself be the final residues of a C-terminal undecapeptide represented by -X^{C}-Tyr-X^{B}-Asn-X^{A}-X-Thr-Arg-X³-Arg-Tyr-C(=O)NR¹R² wherein the sequence -X^{A}-X-Thr-Arg-X³-Arg-Tyr-C(=O)NR¹R² is as discussed in the preceding paragraph, and wherein X^{C} is Arg or Lys and X^{B} is Ile, Leu or Val. An example of such undecapeptide sequences include -Arg-Tyr-Ile-Asn-(Leu or Met)-Leu-Thr-Arg-(Pro or His)-Arg-Tyr-C(=O)NH₂.

Another example of a C terminal undecapeptide sequence which may be present in the agonists with which the invention is concerned is represented by -X^{C}-Tyr-X^{B}-Asn-X^{A}-X-Thr-Arg-X³-Arg-Tyr-C(=O)NR¹R² wherein the sequence -X^{A}-X-Thr-Arg-X³-Arg-Tyr-C(=O)NR¹R² is as discussed in the penultimate paragraph above, X^{C} is His, Asn, or Gln and X^{B} is Ile, Leu or Val. An example of such a sequence is-His-Tyr-(Ile or Leu)-Asn-Leu-(Val/Ile)-Thr-Arg-(Pro or His)-Arg-Tyr-C(=O)NH₂.

In one set of PP-fold mimic agonists with which the invention is concerned, of type (a), or (b) the C-terminal sequence comprising the Y4 receptor-recognition amino acid sequence may be fused at its N-terminus to an amphiphilic amino acid sequence domain comprising at least one alpha helical turn adjacent the N-terminus of the said epitope, and an N-terminal amino sequence of at least two amino acids, the said C- and N-terminal amino acid sequences being joined by peptide bonds to a linker radical, which may be a straight or branched chain alkylene radical, optionally containing one or more double or triple bonds. For example, peptide bonds of the linker radical may be formed with the carboxyl and amino groups respectively of an amino acid of formula NH₂(CH₂)ₙCO₂H wherein n is from 2 to 12, especially 6, 7, 8, 9 or 10. Thus the agonist may be an analogue of NPY, PPY or PP having a Cys-Cys bridge as described, but with amino acid residues corresponding to 5-24 of the native peptide replaced with an aminocarboxylic acid having a carbon chain of from 6 to 10 carbon atoms selected from the group consisting of 6-aminohexanoic acid (epsilon-aminocaproic acid), 7-aminoheptanoic acid, 8-aminooctanoic acid, 9-aminononanic acid, and aminodecanoic acid. In specific embodiments, 8-aminooctanoic acids (herein sometimes abbreviated as "Aoc") are preferred. An example of such an agonist is [Cys2,Aoc5-24,Dcys27]-PP (SEQ ID. No:9).

### N-terminal Y4 receptor-recognition sequences (Type (a) and (b) agonist)

In the N-terminal Y4 receptor-recognition amino acid sequence of the type (a) and (b) agonists of the invention, it is presently preferred that the residue X¹ is Ala, or is absent. Furthermore, in the N-terminal Y4 receptor-recognition amino acid sequence of the agonist, it is currently preferred that the residue X² is Leu, Ile, or Ser.

Specific examples of such N-terminal sequences are H₂N-Ala-Pro-Leu-Glu-, and H₂N- Pro-Leu-Glu-.

Type (a) and (b) Y4 selective agonists with which the invention is concerned may be acylated at their N-terminus to confer resistance to aminopeptidase activity. For example acylation may be with a carbon chain having from 2 to 24 carbon atoms, and N-terminal acetylation is a particular example.

### Specific agonists for use in accordance with the invention

Specific examples of agonists for use in accordance with the invention are the following:
[Cys2,DCys27]-PP (SEQ ID. No:4).
[Lys28,Glu32]PP25-36 (SEQ ID. No:5).
[Glu28,Lys32]PP25-36 (SEQ ID. No:6).
[Cys2,Aoc5-24,Dcys27]-PP (SEQ ID. No:9).
PP2-36 (SEQ ID. No:10).
[His34]-PP (SEQ ID. No:11).
[Ala1,Pro34]-PYY (SEQ ID. No:12).
[Ala2,Pro34]-PYY (SEQ ID. No:13).
[Glu4,Pro34]-PYY (SEQ ID. No:14).
[Arg26,Pro34]-PYY (SEQ ID. No:15).
[Ile28,Pro34]-PYY (SEQ ID. No:16).
[Met30,Pro34]-PYY (SEQ ID. No:17).
[Ala1,Glu4,Pro34]-PYY (SEQ ID No: 25)
[Nle17]PP (SEQ ID. No:32).
[Nle30]PP (SEQ ID. No:33).
[Nle17,Nle30]PP (SEQ ID. No:34).
[Nle17]PP2-36 (SEQ ID. No:37).
[Nle30]PP2-36 (SEQ ID. No:38).
[Nle17,His34]-PP (SEQ ID. No:41).
[Nle30,His34]-PP (SEQ ID. No:42).
[Nle17,Nle30,His34]-PP (SEQ ID. No:43).
[Ala30,Pro34]-PYY (SEQ ID. No:46).
[Leu34]-PP (SEQ ID. No:51).
[Ile34]-PP (SEQ ID. No:52).
[Phe34]-PP (SEQ ID. No:53).
[Lys13]PP2-36 (SEQ ID No: 54) 
S-Cys-Thr-Arg-Pro-Arg-Tyr-CONH2
|
S-Cys-Thr-Arg-Pro-Arg-Tyr-CONH2 (SEQ ID. No:7).
 
S-Cys-Leu-Thr-Arg-Pro-Arg-Tyr-CONH2
|
S-Cys-Leu-Thr-Arg-Pro-Arg-Tyr-CONH2 (SEQ ID. No:18).
 
S-Cys-Leu-Thr-Arg-His-Arg-Tyr-CONH2
|
S-Cys-Leu-Thr-Arg-His-Arg-Tyr-CONH2 (SEQ ID. No:19).
 
S-Cys-Leu-Thr-Arg-Leu-Arg-Tyr-CONH2
|
S-Cys-Leu-Thr-Arg-Leu-Arg-Tyr-CONH2 (SEQ ID. No: 49).
 
S-Cys-Leu-Thr-Arg-Ile-Arg-Tyr-CONH2
|
S-Cys-Leu-Thr-Arg-Ile-Arg-Tyr-CONH2 (SEQ ID. No: 50).
N-Acetyl-PP (SEQ ID. No:30).
N-(N'-hexadecanoyl)-gammaglutamoyl-PP (SEQ ID No: 31).
[N-(N'-hexadecanoyl)-gammaglutamoyl-Lys13,Nle30]PP (SEQ ID No: 35).
[N-(N'-tetradecanoyl)-gammaglutamoyl-Lys13]PP2-36 (SEQ ID No: 39).
[N-(N'-tetradecanoyl)-gammaglutamoyl-Lys13,His34]-PP (SEQ ID No: 44).
[N-(N'-tetradecanoyl)-gammaglutamoyl-Lys13,Ala30,Pro34]-PYY (SEQ ID No: 47).
[Cys2,N-(N'-tetradecanoyl)-gammaglutamoyl-Lys13,DCys27]-PP (SEQ ID No: 48).
[N-(8-(8-gammaglutamoylamino-octanoylamino)-octanoyl)- [Lys13]PP2-36 (SEQ ID No: 55) 
S-Cys-[N-(N'-hexadecanoyl)-gammaglutamoyl]-Lys-Thr-Arg-Leu-Arg-Tyr-CONH2
|
S-Cys-Leu-Thr-Arg-Leu-Arg-Tyr-CONH2 (SEQ ID. No: 29).
[N-{(Ala-Arg-Arg-Arg-Ala-Ala-Arg-Ala)3}-Lys13]PP (SEQ ID No: 36)
[N-{(Ala-Arg-Arg-Arg-Ala-Ala-Arg-Ala)3}-Lys13]PP2-36 (SEQ ID No: 40)
[N-{(Ala-Arg-Arg-Arg-Ala-Ala-Arg-Ala)3}-Lys13,His34]PP (SEQ ID No: 45)
and conservatively substituted analogues thereof.

Particularly preferred Y4-selective agonists for use in accordance with the invention are PP2-36, [His34]-PP (SEQ ID. No:10)., or [Cys2,DCys27]-PP (SEQ ID. No:4). and conservatively substituted analogues thereof.

### Adapted agonists for use in accordance with the invention

Up to this point, basic Y4-selective agonists for use in accordance with the invention have been described generally, and specific examples of such agonists have been provided. However, various modifications may be made to such agonists, including the specifically identified agonists, for the purpose of improving their pharmacokinetics, pharmacodynamics and metabolic properties. Such modifications may involve linking the agonist to functional groupings (also known as motifs) known per se in the art of peptidic or proteinaceous pharmaceuticals. Three particular modifications of particular benefit in the case of the agonists with which the invention is concerned, whether of type (a), (b) or (c) are linkage with serum albumin binding motifs, or a glycosaminoglycan (GAG) binding motifs, or PEGylation.

Also, although PP is excluded from the use in accordance with the invention, the use of PP with such modifications is not excluded.

### Serum-albumin binding motifs

Serum albumin binding motifs are typically lipophilic groups, incorporated to enable a prolonged residence in the body upon administration or for other reasons, which may be coupled in various known ways to peptidic or proteinaceous molecules, for example i) via a covalent linkage to e.g. a functional group present on a side-chain amino acid residue, ii) via a functional group inserted in the peptide or in a suitable derivatized peptide, iii) as an integrated part of the peptide. For example, WO 96/29344 (Novo Nordisk A/S) and P. Kurtzhals et al. 1995 Biochemical J. 312: 725-31, describe a number of suitable lipophilic modifications which can be employed in the case of the agonists with which this invention is concerned.

Suitable lipophilic groups include optionally substituted, saturated or unsaturated, straight or branched hydrocarbon groups of from 10 to 24 carbon atoms. Such groups may form, or may form part of, a side chain to the backbone of the agonist, for example by ether, thioether, amino, ester or amide linkage to a side chain of an amino acid residue in the backbone, or to a backbone carbon or a branch from a backbone carbon of a non-peptidic linker radical in the backbone of a PP-fold mimic agonist. The chemistry strategy for attachment of the lipophilic group is not critical, but the following side chains including lipophilic groups are examples which can be linked to a backbone carbon of the agonist, or suitable branch therefrom:
CH₃(CH₂)ₙCH(COOH)NH-CO(CH₂)₂CONH- wherein n is an integer from 9 to 15,
CH₃(CH₂)ᵣCO-NHCH(COOH)(CH₂)₂CONH- wherein r is an integer form 9 to 15,
CH₃(CH₂)ₛCO-NHCH((CH₂)₂COOH)CONH- wherein s is an integer from 9 to 15,
CH₃(CH₂)ₘCONH-, wherein m is an integer from 8 to 18,
-NHCOCH((CH₂)₂COOH)NH-CO(CH₂)ₚCH₃, wherein p is an integer from 10 to 16,
-NHCO(CH₂)₂CH(COOH)NH-CO(CH₂)_{q}CH₃, wherein q is an integer from 10 to 16,
CH₃(CH₂)ₙCH(COOH)NHCO-, wherein n is an integer from 9 to 15,
CH₃(CH₂)ₚNHCO-, wherein p is an integer from 10 to 18,
-CONHCH(COOH)(CH₂)₄NH-CO(CH₂)ₘCH₃, wherein m is an integer from 8 to 18,
-CONHCH(COOH)(CH₂)₄NH-COCH((CH₂)₂COOH)NH-CO(CH₂)ₚCH₃, wherein p is an integer from 10 to 16,
-CONHCH(COOH)(CH₂)₄NH-CO(CH₂)₂CH(COOH)NH-CO(CH₂)_{q}CH₃, wherein q is an integer from 10 to 16, and
a partly or completely hydrogenated cyclopentanophenanthrene skeleton.

In one chemical synthetic strategy the lipophilic group-containing side chain is a C₁₂, C₁₄, C₁₆ or C₁₈ acyl group, for example a tetradecanoyl group, acylating an amino group present in the side chain of a residue of the backbone of the agonist.

As stated, the modification of agonists for use in accordance to provide improved serum binding characteristics is a strategy which may be applied in general, and particularly in the case of the specific agonists listed above. Thus suitable modified agonists include agonist is [tetradecanoyl-Ala1]-PP (SEQ ID No:20) or [tetradecanoyl-Ala1,His34]-PP (SEQ ID No:21) or a conservatively substituted analogue thereof.

### GAG binding

As in the case of lipophilic serum binding motifs discussed above, the agonists with which this invention are concerned may be modified by incorporation of the GAG binding motif as, or as part of, a side chain to the backbone of the agonist. Known GAG-binding motifs for incorporation in this way include the amino acid sequences XBBXBX and/or XBBBXXBX, wherein B is a basic amino acid residue and X is any amino acid residue. A plurality, for example three, of such sequences may be incorporated in a concatameric (straight chain) or dendrimeric (branched chain) fashion. Specific concatameric GAG motifs include Ala-Arg-Arg-Arg-Ala-Ala-Arg-Ala-Ala-Arg-Arg-Arg-Ala-Ala-Arg-Ala, and Ala-Arg-Arg-Arg-Ala-Ala-Arg-Ala-Ala-Arg-Arg-Arg-Ala-Ala-Arg-Ala-Ala-Arg-Arg-Arg-Ala-Ala-Arg-Ala (both of which may, for example be coupled through an amide bond formed between the C-terminus of the concatameric GAG-binding motif and an amino group in the side chain of a backbone amino acid of the agonist, such as the epsilon amino group of [Lys18,His34]-PP (SEQ ID No:22) or [Lys18]PP (SEQ ID No:23).

Instead of being attached to the agonist as, or as part of a side chain to a backbone residue, the GAG motif may be covalently linked to the C- or (preferably) N-terminus of the agonist, either directly or via a linker radical. Here also the GAG-binding motif may comprise the amino acid sequence XBBXBX and/or XBBBXXBX, wherein B is a basic amino acid residue and X is any amino acid residue, for example the sequence [XBBBXXBX]ₙ where n is 1 to 5, B is a basic amino acid residue and X is any amino acid residue. Such concatameric repeats tend to form alpha helices when they bind to GAG's, and consequently when fused to the C-terminal hexapeptide/last alpha helical turn, can stabilise that turn and thereby present the combined structure in an optimal way for Y4 receptor recognition. Specific examples of agonists of this type are [XBBBXXBX-XBBBXXBX]PP or [XBBBXXBX-XBBBXXBX-XBBBXXBX]PP, wherein B is a basic amino acid residue and X is any amino acid residue, particularly Ala-Arg-Arg-Arg-Ala-Ala-Arg-Ala-Ala-Arg-Arg-Arg-Ala-Ala-Arg-Ala-Ala-Arg-Arg-Arg-Ala-Ala-Arg-Ala-PP (SEQ ID No:24).

The Y4 selective agonists with which the present invention is concerned are useful, inter alia, in indications for which prolonged exposure is desirable. For such indications in particular, the agonists preferably comprise a glycosamino glycan (GAG) binding motif as discussed above. Such motifs ensure that the agonists bind to GAGs in the extracellular matrix, and thereby ensures prolonged local exposure of the Y4 receptors in that tissue. Growth factors, chemokines etc bind to GAGs through patches of basic amino acids, which interact with the acidic sugars of the GAGs. These positively charged epitopes on the growth factors are usually composed of side chains from basic residues, which are not necessarily located consecutively in sequence but are often presented in close proximity by a secondary structural element such as an a-helix or a turn or by the overall three dimensional structure of the protein. Certain GAG-binding, linear sequences, discussed above, have been described, for example XBBXBX and XBBBXXBX where B represents a basic residue (Hileman et al. Bioassays 1998, 20: 156-67). These segments have been shown by circular dichroism to form α-helices upon binding to GAGs. If such sequences are placed for example in a concatameric or dendrimeric construct where for example three such sequences are presented - for example each as a ARRRAARA sequence - the resulting 24-mer peptide - for example ARRRAARA-ARRRAARA-ARRRAARA - ensures a retention in the extracellular matrix similar to high molecular weight polylysine, i.e. it is not washed out during a 4 hour perfusion period (Sakharov et al. FEBS Lett 2003, 27: 6-10).

Thus Growth factors and chemokines are naturally constructed with two types of binding motifs: one binding motif for the receptor through which signal transduction is achieved and one binding motif for GAG's through which attachment and long-lasting local activity is achieved. Peptides such as PYY and NPY are neuropeptides and hormones, which are rather rapidly washed out of the tissue and are not optimized for long-lasting local activity. By attaching a GAG-binding motif to a Y4 selective agonist according to the present invention or to the classical known peptide agonist PYY3-36 - a bi-functional molecule similar to the growth factors and chemokines is constructed having both a receptor binding epitope in the PP-fold peptide part and a GAG-binding motif.

As discussed already above, in a preferred embodiment of the invention, a GAG-binding motif, for example a concatameric 24-mer sequence as described above, is placed as an N-terminal extension of the Y4-selecive agonist. This placement is particularly interesting for N-terminally truncated PP-fold mimic agonists. Although those described herein are Y4 selective, they may show a somewhat decreased affinity or potency for the Y4 receptor as compared to native PP. With such truncated PP-fold mimic agonists, it can be an advantage to extend with a concatameric GAG-binding motif, since this upon binding to GAGs' will form an α-helix which will be able to help stabilize the helical part and thereby help present the far C-terminal segment in the correct manner.

Another suitable position for introduction of a GAG binding motif - such as a basic concatameric or dendrimeric construct - of the PP-fold peptides is for NPY peptides and analogs thereof, position 14, and for PYY and PP peptides and analogs thereof position 13. However, as discussed above, a residue comprising the GAG-binding motif can be introduced at any position in the agonists provided that this introduction is-consistent with retention of the PP-fold structure required in the type (a) and (b) agonists with which the invention is concerned, and the required C-terminal Y2-recognition sequence. Thus, a GAG-binding motif could be placed as part of the spacer construct in the analogs in which part of the PP-fold is replaced by a non-peptide spacer.

### PEGylation

In PEGylation, a polyalkyleneoxide radical or radicals, is/are covalently coupled to peptidic or proteinaceous drugs to improve effective half life in the body following administration. The term derives from the preferred polyalkyleneoxide used in such processes, namely that derived from ethylene glycol - polyethyleneglycol, or "PEG".

A suitable PEG radical may be attached to the agonist by any convenient chemistry, for example via a backbone amino acid residue of the agonist. For instance, for a molecule like e.g. PEG, a frequently used attachment group is the epsilon-amino group of lysine or the N-terminal amino group. Other attachment groups include a free carboxylic acid group (e.g. that of the C-terminal amino acid residue or of an aspartic acid or glutamic acid residue), suitably activated carbonyl groups, mercapto groups (e.g. that of a cysteine residue), aromatic acid residues (e.g. Phe, Tyr, Trp), hydroxy groups (e.g. that of Ser, Thr or OH-Lys), guanidine (e.g. Arg), imidazole (e.g. His), and oxidized carbohydrate moieties.

When the agonist is PEGylated it usually comprises from 1 to 5 polyethylene glycol (PEG) molecules such as, e.g. 1, 2 or 3 PEG molecules. Each PEG molecule may have a molecular weight of from about 5 kDa (kiloDalton) to about 100 kDa, such as a molecular weight of from about 10 kDa to about 40 kDa, e.g., about 12 kDa or preferably no more than about 20 kDa.

Suitable PEG molecules are available from Shearwater Polymers, Inc. and Enzon, Inc. and may be selected from SS-PEG, NPC-PEG, aldehyde-PEG, mPEG-SPA, mPEG-SCM, mPEG-BTC, SC-PEG, tresylated mPEG (US 5,880,255), or oxycarbonyl-oxy-N-dicarboxyimide-PEG (US 5,122,614).

### Serum albumin, GAG and PEG

Whether the modification to the agonist is attachment of a group to facilitate serum binding, GAG binding or improved stability via PEGylation, the serum albumin binding motif or GAG binding motif, or PEG radical may be, or may form part of, a side chain of a backbone carbon of the agonist corresponding to any of the following positions of PYY or PP: 1, 3, 4, 6, 7, 10, 11, 12, 13, 15, 16, 17, 18, 19, 21, 22, 23, 25, 26, 28, 29, 30 and 32, or corresponding to any of the following positions of NPY: 1, 3, 4, 6, 7, 10, 11, 12, 14, 15, 16, 17, 18, 19, 21, 22, 23, 25, 26, 28, 29, 30 and 32.

Particularly when the agonist is of type (b), the serum albumin binding motif or GAG binding motif, or PEG radical may also be, or may form part of part of, a side chain of a backbone carbon corresponding to any of the following positions of PYY, NPY or PP: 2, 5, 8, 9, 13, 14, 20, and 24.

### Conjugation to larger biomolecules

As described above, It is possible to link certain motifs at various positions in the PP-fold peptide or PP-fold peptide mimic without impairing their high Y receptor affinity (see examples). In a similar way the selective Y4 receptor agonists may be used as fusion proteins where they are linked for example to albumin or another protein or carrier molecule which provides beneficial pharmacokinetic or other types of properties such as for example decreased renal elimination. There are multiple chemical modifications and linkers which can be used for such a covalent attachment as known in the art, just as there are multiple proteins or carriers which can be used. Especially covalent attachment of the selective Y4 peptide agonist to albumin is preferred and at one of the positions in the PP-fold structure, which have been pointed out elsewhere herein in relation to modifications with the various motifs. Such fusion proteins can be produced through various semi-synthetic techniques where the peptide may be made through peptide synthesis as described herein and the biomolecule through recombinant technology. The fusion protein may also be made enteriely as a recombinant molecule expressed for example as a precursor molecule extended by a Gly-Lys-Arg sequence, which when expressed as a secretory protein in eukaryotic cells will be cleaved by biosynthetic enzymes and the Gly turned into the carboxyamide on the C-terminal Tyr residue of the C-terminal Y4 receptor recognition sequence.

### Stabilization

As mentioned at various points herein, many of the of the selective Y4 agonists of the present invention are stabilized in various ways, for example by N-terminal acylation, or by replacement of parts of the agonist backbone with a non-peptidic linker, or by internal cyclizing cross links to stabilize the PP-fold structure. This stabilization in most cases serves two purposes, one being to present the receptor recognition epitopes in an optimal way for the Y4 receptors by preserving the PP-fold; is the other being to stabilize the peptide against degradation, i.e. especially proteolytic degradation. This is particularly important when the selective Y4 agonists are also modified to obtain prolonged half-life, sustained release, and/or long-lasting tissue exposure, by the attachment of the various motifs discussed above. Normally, the peptide agonists will be relatively rapidly eliminated mainly through the kidneys and the protein stability as such may not be a critical issue; however, when the presence of the peptide in various body fluids in one or more ways is prolonged for many hours it is particularly important that the biological activity of the peptide is also kept intact, i.e. that it is stabilized in a form resistant to proteolytic attacks, as described both in general and in relation to the specific examples of selective Y4 agonist peptides, for example the PP-fold stabilized, cyclic [Cys2,D-Cys27]PP and the various analogs of this.Thus in preferred embodiments of the invention the Y4 selective agonists are both structurally stabilized - through structural alterations some of which are specifically indicated in the examples presented below - and they are decorated with various motifs and/or fused to biomolecules and/or prepared in pharmaceutical manners to obtain sustained release etc. to obtain prolonged receptor exposure.

### Helix Inducing Peptides

Acylation of the N-terminus of the agonists with which the invention is concerned has been mentioned as a means of stabilising the agonist against the action of aminopeptidases. Another stabilising modification involves the covalent attachment of a stabilizing peptide sequence of 4-20 amino acid residues covalently at the N-and/or the C-terminus, preferably the N-terminus. The amino acid residues in such a peptide are selected from the group consisting of Ala, Leu, Ser, Thr, Tyr, Asn, Gln, Asp, Glu, Lys, Arg, His, Met and the like. In an interesting embodiment the N-terminal peptide attachment comprises 4, 5 or 6 Lys residues, for example Lys-Lys-Lys-Lys-Lys-Lys-PP or Lys-Lys-Lys-Lys-Lys-Lys-[His34]PP These can be linked at the N-terminus of the PP-fold peptide agonist or they may be placed at the N-terminus of an N-terminally truncated PP-fold mimic agonist or an N-terminally shortened PP-fold mimic agonist, where a spacer peptide has been introduced between the new N-terminus an the stabilizing peptide. Such modifications are particularly interesting since a string of Lys residues will have a tendency to form an alpha helical structure and induce this in the subsequent residues, which as discussed above is beneficial for the receptor presentation of the last helical turn and the C-terminal Y2 receptor recognition amino acid sequence of the selective Y2 agonist peptides of the present invention. A general description of such stabilizing peptide extensions is given in WO 99/46283 (Zealand Pharmaceuticals), which is hereby incorporated by reference.

The receptor agonists with which the invention is concerned may be prepared by well-known methods such as, e.g., a synthetic, semisynthetic and/or recombinant method. The methods include standard peptide preparation techniques such as, e.g., solution synthesis, and solid-phase synthesis. Based on textbook and general knowledge within the field, a person skilled in the art knows how to proceed in order to obtain the agonists and derivatives or modifications thereof.

In the following, the specific agonists according to the invention are described in relation to their molecular pharmacological properties as well as peptide chemical and stability properties.

### N-Acetyl-PP (SEQ ID. No:30), N-(N'-hexadecanoyl)-gammaglutamoyl-PP (SEQ ID No: 31), [Nle17]PP (SEQ ID. No:32), [Nle30]PP (SEQ ID. No:33). [Nle17,Nle30]PP (SEQ ID. No:34), [N-(N'-hexadecanoyl)-gammaglutamoyl-Lys13,Nle30]PP (SEQ ID No: 35), [N-((Ala-Arg-Arg-Arg-Ala-Ala-Arg-Ala)3)-Lys13]PP (SEQ ID No: 36)

These peptides illustrate useful variants or analogs of the endogenous Y4 agonist PP. N-Acetyl-PP and N-(N'-hexadecanoyl)-gammaglutamoyl-PP are analogs of PP which have been modified at the alpha-amino group providing protection against proteolytic degradation by amino peptidases. In the case of N-(N'-hexadecanoyl)-gammaglutamoyl-PP the N'-hexadecanoyl)-gammaglutamoyl motif at the same time constitute a serum albumin binding motif which secures a prolonged effective T½. [Nle17]PP, [Nle30]PP, [Nle17,Nle30]PP represent analogs in which Met residues in PP2-36 have been substituted with a non-oxidizable in this case non-natural residue norleucine (Nle). Due to the high structural similarity between Met and Nle this substitution does not alter the high Y4 receptor affinity and selectivity. [N-(N'-tetradecanoyl)-gammaglutamoyl-Lys13]-PP and [N-{(Ala-Arg-Arg-Arg-Ala-Ala-Arg-Ala)3}-Lys13]PP represent analogs of PP in which motifs have been attached in order to obtain binding to serum albumin etc. and thereby a prolonged effective T½ and binding to GAG's and thereby slow release from the injection site and/or long-lasting local Y4 receptor exposure due to prolonged presence in the tissue - in both cases the motifs have been attached to an introduced Lys residue at position 17. Due to the fact that the Lys residue - and thereby also the various motifs - is introduced far away in the structure from where the peptides are recognized by the Y receptors these modifications do not affect the high Y4 affinity and selectivity of the basic peptide. As presented elsewhere herein another useful substitution could be for example a PEGylation. These analogs of PP are members of one group - in this case where the basic PP structure has been preserved except in the cases where attachment site(s) for various motifs have been intrduced - out of many different groups of highly selective Y4 over Y1 and Y2 agonists described in the present invention from which compounds can be selected for treatment of diseases responsive to Y4 agonist such as obesity, secretory diarrhea, irritable bowl disease etc. These are all novel compounds.

### PP2-36 (SEQ ID No: 10)

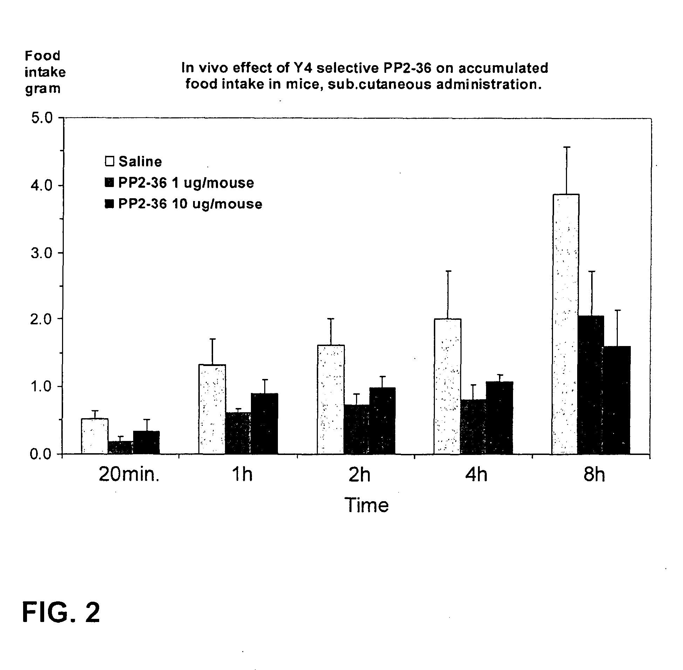

This peptide is a type (a) which represents PP analogs with N-terminal Y4 receptor recognition amino acid sequences where residue number 1 is absent. PP2-36 is a highly selective Y4 receptor agonist with improved stability as compared to PP1-36. PP is normally a substrate for DPP-IV degradation as it has a Pro in position 2. Thus, by removing the first residue from PP a peptide is generated where the PP fold structure is not jeopardized because residue 1 (Ala1) does not make any interactions with the anti-parallel helix (this is taken care of by Pro2) and because PP2-36 no longer like the wild-type peptide is a substrate for DPP-IV. Importantly, the Y4 receptor is not dependent on the presence of a residue in position 1, in contrast the Y1 receptor is dependent on the presence of a residue in position 1. Thus, PP2-36 is both a more stable peptide and it is a selective Y4 receptor agonist which has an improved selectivity window towards the Y1 receptor.
*Receptor recognition profile -* PP2-36 binds with an affinity to the Y4 receptor, IC50 = 0.64 nM, which is similar to the affinity of PP, IC50 = 0.41. The affinity of PP2-36 on the Y1 and Y2 receptors is > 1000 nM (Table 1). PP2-36 acts as an agonist on the Y4 receptor with a potency of 0.64 nM (EC50) which also is very similar to the potency of PP (EC50 = 0.85 nM) as measure in the inositol phosphate turnover assay performed in transfected COS-7 cells as described in the experimental section (Table 2). Both PP2-36 and PP are low potency ligands for the Y2 receptor, i.e. with EC50 values > 1000 nM. However, on the Y1 receptor PP2-36 has a potency which is 3-4 fold lower than that of PP, EC50 values being 297 versus 83 nM (Table 2). Thus PP2-36 is a highly selective Y4 agonist with > 300 fold window to both the Y1 and the Y2 receptor as determined in a functional in vitro assay.
*Protein stability -* PP2-36 has the advantage as compared to PP1-36 that it is more stable and therefore a better biopharmaceutical agent as it is not a substrate for DPP-IV - as PP is due to the presence of a Pro residue in position 2 - and since the novel N-terminal Pro residue in PP2-36 prevents against degradation by most other amino peptidases. These stability properties are particularly useful in the context where a prolonged presence of the peptide in body fluids is obtained through various means such as motif modifications of the peptide as such or through various forms of pharmaceutical methods such as sustained release formulations.
*In vivo effect on acute food intake -* Either saline or PP2-36 was administered by single subcutaneous injections to groups of 8 mice in doses of 1 and 10 µg per animal after 16 hours of fasting. In Fig. 2 is shown the accumulated food intake of the mice during 8 hours. The selective Y4 receptor agonist, PP2-36 inhibited food intake during the first 20 minutes after initiation of the meal to approx. 50 % of that ingested by the group of animals receiving saline. The 50 % suppressive effect on food intake was observed also when this was measured during the first full hour. The effect of 1 ug and 10 ug of PP2-36 was rather similar. The suppressive effect of PP2-36 was sustained for the full period of 8 hours after the single sub cutaneous injection (Fig 2). Thus PP2-36, which is highly selective for the Y4 over the Y2 and Y1 receptors is an efficient suppressor of food intake in mice as previously demonstrated for PP (Asakawa et al. 1999 Peptides 20: 1445-48, Asakawa 2003 Gastroenterology 124: 1325-36). It has previously been demonstrated that this effect of PP is also observed in normal human subjects (Batterham et al. 2003 Clin.Endocrinol.Metab. 88: 3989-92) as well as in mobidly obese patients (Berntson et al. 1993 Peptides 14: 497-503).

### [Nle17]PP2-36 (SEQ ID. No:37), [Nle30]PP2-36 (SEQ ID. No:38), [N-(N'-tetradecanoyl)-gammaglutamoyl-Lys13]PP2-36 (SEQ ID No: 39), [N-{(Ala-Arg-Arg-Arg-Ala-Ala-Arg-Ala)3}-Lys13]PP2-36 (SEQ ID No: 40)

These peptides illustrate useful variants or analogs of one example of a highly selective Y4 receptor agonists of type (a) PP2-36. [Nle17]PP2-36 and [Nle30]PP2-36 represent analogs in which Met residues in PP2-36 have been substituted with a non-oxidizable in this case non-natural residue norleucine (Nle). Due to the high structural similarity between Met and Nle this substitution does not alter the high Y4 receptor affinity and selectivity. [N-(N'-tetradecanoyl)-gammaglutamoyl-Lys13]-PP2-36 and [N-{(Ala-Arg-Arg-Arg-Ala-Ala-Arg-Ala)3}-Lys13]PP2-36 represent analogs of PP2-36 in which motifs have been attached in order to obtain binding to serum albumin etc. and thereby a prolonged effective T½ and binding to GAG's and thereby slow release from the injection site and/or long-lasting local Y4 receptor exposure due to prolonged presence in the tissue - in both cases the motifs have been attached to an introduced Lys residue at position 17. Due to the fact that the Lys residue - and thereby also the various motifs - is introduced far away in the structure from where the peptides are recognized by the Y receptors these modifications do not affect the high Y4 affinity and selectivity of the basic peptide. As presented elsewhere herein another useful substitution could be for example a PEGylation. These analogs of PP2-36 are members of one group out of many different groups of highly selective Y4 over Y1 and Y2 agonists described in the present invention from which compounds can be selected for treatment of diseases responsive to Y4 agonist such as obesity, secretory diarrhea, irritable bowl disease etc. These are all novel compounds.

### [His34]-PP (SEQ ID No: 11), [Leu34]-PP (SEQ ID. No:51), [Ile34]-PP (SEQ ID. No:52), [Phe34]-PP (SEQ ID. No:53).

These peptides represent a group of type (a) highly selective Y4 receptor agonist being PP analogs having a substitutions in the C-terminal Y4 receptor recognition amino acid sequence, in these preferred cases a single substitution in position 34, which normally is a Pro residue. The postion-34 substituted PP analogs constitute one of many different groups of highly selective Y4 over Y1 and Y2 agonists described in the present invention from which compounds can be selected fr treatment of diseases responsive to Y4 agonist such as obesity, secretory diarrhea, irritable bowl disease etc. These are novel compounds.
*Receptor recognition profile -* As shown in Table 1 substitution of the natural Pro34 with another residue exemplified by [His34]PP does not impair the affinity for the Y4 receptor as [His34]PP binds with high affinity to the Y4 receptor (IC50 = 0.48 nM) which is similar to the affinity of PP (IC50 = 0.41 nM). Importantly, the affinity of [His34]PP is not measurable on the Y1 and Y2 receptors (IC50 > 1000 nM). Thus it is a highly selective ligand for the Y4 receptor as opposed to the Y1 and Y2 receptors. [His34]-PP acts as an agonist on the Y4 receptor with a potency of 1.2 nM (EC50) which is very similar to the potency of PP (EC50 = 0.64 nM) as measure in the inositol phosphate turnover assay (Table 2). As PP, [His34]-PP displays low potency for the Y2 receptor, i.e. an EC50 value > 1000 nM. However, on the Y1 receptor [His34]-PP has a potency which is far lower than that of PP, EC50 values being > 1000 nM for [His34]-PP versus 83 nM for PP (Table 2). Thus [His34]-PP is a highly selective Y4 agonist with > 1000 fold window to both the Y1 and the Y2 receptor as determined in a functional in vitro.
*Protein stability -* [His34]-PP has a protein stability similar to that of PP.

### [Nle17,His34]-PP (SEQ ID. No:41), [Nle30,His34]-PP (SEQ ID. No:42). [Nle17,Nle30,His34]-PP (SEQ ID. No:43), [N-(N'-tetradecanoyl)-gammaglutamoyl-Lys13,His34]-PP (SEQ ID No: 44), [N-{(Ala-Arg-Arg-Arg-Ala-Ala-Arg-Ala)3}-Lys13,His34]PP (SEQ ID No: 45)

These peptides illustrate useful variants or analogs of one example of highly selective Y4 receptor agonists of type (a) characterized by substitution in the C-terminal Y4 recognition amino acid sequence, in this case position 34 and in this particular case being [His34]PP. [Nle17,His34]-PP, [Nle30,His34]-PP, and [Nle17,Nle30,His34]-PP represent analogs in which one or both of the Met residues in [His34]PP have been substituted with a non-oxidizable in this case non-natural residue norleucine (Nle). Due to the high structural similarity between Met and Nle this substitution does not alter the high Y4 receptor affinity and selectivity. [N-(N'-tetradecanoyl)-gammaglutamoyl-Lys13,His34]-PP and [N-{(Ala-Arg-Arg-Arg-Ala-Ala-Arg-Ala)3}-Lys13,His34]PP represent analogs of [His34]PP in which motifs have been attached in order to obtain binding to serum albumin etc. and thereby a prolonged effective T½ and binding to GAG's and thereby slow release from the injection site and/or long-lasting local Y4 receptor exposure due to prolonged presence in the tissue - in both cases the motifs have been attached to an introduced Lys residue at postion 17. Due to the fact that the Lys residue - and thereby also the various motifs - is introduced far away in the structure from where the peptides are recognized by the Y receptors these modifications do not affect the high Y4 affnity and selectivity of the basic peptide. As presented elsewhere herein another useful substitution could be for example a PEGylation. These analogs of [His34]PP are members of one group out of many different groups of highly selective Y4 over Y1 and Y2 agonists described in the present invention from which compounds can be selected for treatment of diseases responsive to Y4 agonist such as obesity, secretory diarrhea, irritable bowl disease etc.These are all novel compounds.

### [Ala1,Pro34]-PYY (SEQ ID No: 12), [Ala2,Pro34]-PYY (SEQ ID No: 13), [Glu4,Pro34]-PYY (SEQ ID No: 14), [Ala1,G1u4,Pro34]-PYY (SEQ ID No: 25)

These peptides are type (a) selective Y4 receptor agonists which represents PYY analogs in which a C-terminal Y4 receptor amino acid sequence has been introduced through the Pro34 substitution in the C-terminal end of PYY and in which the selectivity towards the Y1 receptor has been increased through a substitution(s) in the N-terminal Y4 receptor recognition amino acid sequence, i.e either the Ala1 for Tyr substitution, the Ala2 for Pro substitution or the Glu4 for Lys substitution. In [Ala1,Glu4,Pro34]-PYY, two substitutions have been made in the N-terminal segment. [DesTyr1,Pro34]-PYY is another peptide, which also increases the selectivity of the peptide towards the Y1 receptor. These analogs of [Pro34]PYY are members of one group out of many different groups of highly selective Y4 over Y1 and Y2 agonists described in the present invention from which compounds can be selected for treatment of diseases responsive to Y4 agonist such as obesity, secretory diarrhea, irritable bowl disease etc.These peptides are all novel compounds.
*Receptor recognition profile* - As examples, [Ala1,Pro34]-PYY binds with high affinity to the Y4 receptor (IC50 = 4.4 nM) as opposed to the Y2 receptor (IC50 > 1000 nM) and the Y1 receptor (IC50 = 176 nM) (Table 1). Thus, the Ala1 and Pro34 substitutions in PYY have increased the affinity on the Y4 receptor from 30 to 4.4 nM and decreased the affinity on the Y1 receptor from 16 to 176 nM and on the Y2 receptor from 0.22 nM to >1000 nM. The receptor recognition profile for, for example [Glu4,Pro34]-PYY is rather similar with the affinity on the Y4 receptor being 2.4 nM and on the Y1 being 99 nM (Table 1).

### [Arg26,Pro34]-PYY (SEQ ID No: 15), [Ile28,Pro34]-PYY (SEQ ID No: 16), [Met30,Pro34]-PYY (SEQ ID No: 17)

These peptides are also type (a) selective Y4 receptor agonists representing PYY analogs in which a C-terminal Y4 receptor amino acid sequence has been introduced through the Pro34 substitution in the C-terminal end of PYY and in which the selectivity towards the Y1 receptor has been increased through a single substitution in the C-terminal segment of the PYY sequence, i.e either the Arg26 for His substitution, the Ile28 for Leu substitution or the Met30 for Leu substitution. These analogs of [Pro34]PYY are members of one group out of many different groups of highly selective Y4 over Y1 and Y2 agonists described in the present invention from which compounds can be selected for treatment of diseases responsive to Y4 agonist such as obesity, secretory diarrhea, irritable bowl disease etc.These are all novel compounds.
*Receptor recognition profile* - As examples, [Arg26,Pro34]-PYY binds with high affinity to the Y4 receptor (IC50 = 0.43 nM) as opposed to the Y2 receptor (IC50 = 177 nM) and the Y1 receptor (IC50 = 15 nM) (Table 1). The receptor recognition profile for, for example [Ala30,Pro34]-PYY is rather similar but with an even bigger selectivity window to the Y1 receptor as its affinity on the Y4 receptor is 0.99 nM and on the Y1 being 173 nM and on the Y2 receptor > 1000 nM. The corresponding data for a third example, [Met30,Pro34]PYY are presented in Table 1.

### [Cys2,D-Cys27]PP (SEQ ID No: 4)

This peptide represent a type (b) highly selective Y4 agonist, which has been designed both for receptor selectivity and for high stability. [Cys2,D-Cys27]PP represent a group of selective Y4 agonists in which a linker, in this case an intra-molecular disulfide bridge has been introduced between in this particular case a Cys substituted for Pro in positions 2 and a D-Cys substituted for Tyr in position 27 in the full length PP molecule in order to stabilize its PP-fold structure. [Cys2,D-Cys27]PP is also protected against degradation by DPP-IV as it no longer has Pro in position 2. This peptide is active also with the N-terminus acetylated a modification which further prevents the degradation of the peptide by aminopeptidases. Both the free peptide and for example the acetylated version are novel compounds.
*Receptor recognition profile-* [Cys2,D-Cys27]PP binds with single digit nanomolar affinity to the Y4 receptor, IC50 = 8.2 nM. It is highly selective for the Y4 receptor as the binding affinity of [Cys2,D-Cys27]PP on the Y1 and Y2 receptors is > 1000 nM (Table 1). [Cys2,D-Cys27]PP acts as an agonist on the Y4 receptor with a potency of 4.6 nM (EC50) only 7-fold lower than that of PP (EC50 = 0.64 nM) as measure in the inositol phosphate turnover assay performed in transfected COS-7 cells (Table 2). [Cys2,D-Cys27]PP is a low potency ligands for the Y2 receptor, i.e. with and EC50 value > 1000 nM. Importantly, in contrast to PP the potency of [Cys2,D-Cys27]PP is very low on the Y1 receptor i.e. the EC50 value for PP is 83 nM and unmeasurably low for [Cys2,D-Cys27]PP (Table 2). Thus [Cys2,D-Cys27]PP is a highly selective Y4 agonist with an improved selectivity window to both the Y1 and the Y2 receptor. *Protein stability -* [Cys2,D-Cys27]PP has the advantage as compared to PP that it is more stable and therefore a better biopharmaceutical agent as it is not a substrate for DPP-IV and because its PP-fold has been stabilized by an intra-molecular disulfide bridge. These stability properties are particularly useful in the context where a prolonged presence of the peptide in body fluids is obtained through various means such as motif modifications of the peptide as such or through various forms of pharmaceutical methods such as sustained release formulations.

### [Cys2,N-(N'tetradecanoyl)-gammaglutamoyl-Lys13,DCys27]-PP (SEQ ID No: 48).

This peptide represent a group of type (b) highly selective Y4 agonist, which has been designed both for receptor selectivity and for high stability through an intra-molecular linker as described above and has been modified by attachment of a binding motif in this case exemplified with a serum albumin binding motif attached to an introduced Lys13 residue in [Cys2,D-Cys27]PP. It has binding Y receptor properties similar to those of [Cys2,D-Cys27]PP due to the position of the attachment site.

### [Cys2,Aoc5-24,DCys27]-PP (SEQ ID No: 9)

[Cys2, Aoc5-24,D-Cys27]PP is a type (b) selective Y4 agonist which represents centrally truncated disulfide stabilized PP peptide mimics which are selective for the Y4 receptor and which have the advantage over PP that they are much smaller - only 16 residues as compared to 36 residues - and stabile. [Cys2,Aoc5-24,DCys27]-PP is a novel compound.
*Receptor recognition profile -* [Cys2, Aoc5-24,D-Cys27]PP binds with an affinity of 286 nM to the Y4 receptor and with unmeasurable affinity to the Y1 and Y2 receptors (Table 1)

### [Lys28,G1u32]PP25-36 (SEQ ID No: 5) and [Glu28,Lys32]PP25-36 (SEQ ID No: 6)

These are type (b) selective Y4 receptor agonists which represent cyclic PP analogs stabilized by an intramolecular lactam bridge. In these cases. Ile28 of PP (position 4 in the actual dodecapeptide) is substituted with a Lys or Glu residue and Thr32 (position 8 in the actual dodecapeptide) is substituted with a Glu or Lys residue and the amino and carboxylic groups in the side chains of these residues in positions 28 and 32 have been joined by lactamization. These peptides are novel compounds.

S-Cys-Thr-Arg-Pro-Arg-Tyr-CONH2
|
S-Cys-Thr-Arg-Pro-Arg-Tyr-CONH2 (SEQ ID No: 7)

S-Cys-Leu-Thr-Arg-Pro-Arg-Tyr-CONH₂
|
S-Cys-Leu-Thr-Arg-Pro-Arg-Tyr-CONH₂ (SEQ ID No: 18)

S-Cys-Leu-Thr-Arg-Pro-Arg-Tyr-CONH2
|
S-Cys-Leu-Thr-Arg-His-Arg-Tyr-CONH2 (SEQ ID No: 26)

S-Cys-Leu-Thr-Arg-Leu-Arg-Tyr-CONH2
|
S-Cys-Leu-Thr-Arg-Leu-Arg-Tyr-CONH2 (SEQ ID. No: 49).

S-Cys-Leu-Thr-Arg-Ile-Arg-Tyr-CONH2
|
S-Cys-Leu-Thr-Arg-Ile-Arg-Tyr-CONH2 (SEQ ID. No: 50).

These are type (c) selective Y4 receptor agonists which represent dimeric constructs in which two C-terminal Y4 receptor recognition amino acid sequences have been joined, in these cases by a disulfide bridge between two Cys residues. These have the advantage that they are small but high potency, selective Y4 receptor ligands. These are novel compounds.
*Receptor recognition profile -* As examples, the [CTRPRY-amide]x2 homo-dimer (SEQ ID No: 18) binds with an affinity of 29 nM to the Y4 receptor and with un measurable affinity to the Y1 and Y2 receptors (Table1), whereas the longer [CLTRPRY-amide]x2 homo-dimer (SEQ ID No: 26) binds with even higher affinity to the Y4 receptor (IC50 = 1.3 nM) and again with unmeasurable affinity to the Y1 and Y2 receptors (Table 1). Thus this type (c) compounds have a large selectivity window between the high affinity Y4 binding and low affinity Y1 and Y2 receptor binding - i.e. a > 1000 fold difference for the [CLTRPRY-amide]x2 homo-dimer example presented here.

S-Cys-[N-(N'-hexadecanoyl)-gammaglutamoyl]-Lys-Thr-Arg-Leu-Arg-Tyr-CONH2
|
S-Cys-Leu-Thr-Arg-Leu-Arg-Tyr-CONH2 (SEQ ID. No: 29).

This peptide represent a group of type (c) selective Y4 receptor compounds which are dimeric constructs in which two C-terminal Y4 receptor recognition amino acid sequences have been joined, in these cases by a disulfide bridge between two Cys residues, and which have been decorated by a binding motif - in this case a serum albumin binding motif attached to a Lys for Leu substitution at position "31 ". Thus despite the small size of the basic peptide this type of Y4 selective compounds are not rapidly eliminated in the kidney and consequently have a prolonged T½. The attachment site for the binding motif has been placed close to the disulfide linker, where it has minimal effect on Y4 receptor recognition as exemplified with the high affinity binding of the shorter [CTRPRY-amide]x2 above which is missing this position "31" residue.

### Clinical Indications

The Y4-specific agonists with which the invention is concerned are of value in the treatment of conditions responsive to activation of Y4 receptors. Such conditions include those for which regulation of energy intake or energy metabolism is indicated. For any such use, the agonist may be one which comprises a modification or motif which confers stability towards peptidases, serum protein binding properties, PEGylation or GAG-binding motif to prolong serum and / or tissue half-life.

Diseases or conditions in which regulation of energy intake or energy metabolism is indicated include obesity and overweight, and conditions in which obesity and overweight are considered contributory factors, such as bulimia, bulimia nervosa, Syndrome X (metabolic syndrome), diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, coronary artery disease, myocardial infarction, peripheral vascular disease. stroke, thromboembolic diseases, hypercholesterolemia, hyperlipidemia, gallbladder disease, osteoarthritis, sleep apnea, reproductive disorders such as polycystic ovarian syndrome, or cancer of the breast, prostate, or colon.

Y4 selective agonists are also of value in the treatment of diarrhoea or hyper-secretion from intestinal stomia, and in the treatment of nausea or emesis, or as anti-nausea or antiemetic agents or co-treatment with drugs prone to cause nausea and/or emesis.

### 1. Obesity and Overweight

It was suggested already in the seventies that PP might be involved in the control of food intake. Recently, much evidence from rodent studies has accumulated showing very clearly that PP is in fact a powerful and efficient anorexigenic peptide when administered peripherally (Asakawa et al. Peptides 1999, 20; 1445-8; Katsuura et al. Peptides 2002, 23: 323-9; Asakawa et al. Gastroenterology 2003, 124: 1325-36). Since PP has no effect on appetite, food intake etc. in Y4 knock out animals it is very likely that PP acts through the Y4 receptor to reduce appetite and food intake (Batterham et al. 2004 abstract S3.3 from International NPY symposium in Coimbra Portugal). PP also has effect on food intake in diet induced obese animals. PP receptors have been found especially in the brain stem in area postreama and on vagal motor neurones where the blood brain barrier is not efficient and where circulating hormones such as PP can get access to the neurones. Thus it is very likely that the Y4 receptors in the NTS in the brain stem are a major target through which PP acts to suppress appetite and food intake. However, recent evidence also points to the possibility that PP may also act through Y receptors in the arcuate nucleus conceivably on the POMC and perhaps also the NPY/ AgRP neurones (Batterham et al. Coimbra NPY meeting abstract S3.3). Low levels of PP are found in obese subjects especially Prader-Willi syndrome (Zipf et al. J.C.E.M.1981, 52: 1264-6, Holst et al 1983, Int.J.Obes. 7: 529-38, Glaser et al Horm.Metab. 1988, 20: 288-92) and high PP levels are found in patients with anorexia nervosa. Importantly, infusion of PP in man decreases appetite and food intake for up to 24 hours (Batterham et al. JCEM 2003, 88: 3989-92). Thus, the effect of PP on food intake was observed after the PP levels in the circulation had returned to normal levels. Such long lasting effects on appetite etc, is well know from other compounds for example also from ICV injection of AgRP. Importantly infusion of PP has been shown to decrease food intake in mobidly obese patients with Prader Willi syndrome (Berntson et al 1993 Peptides 14: 497-503).

Hence, the Y4 selective agonists with which the invention is concerned are suitable for use in a subject, such as a mammal including a human, in order to regulate the energy intake. Accordingly, the invention relates to methods for altering energy intake, food intake, appetite, and energy expenditure. A method is disclosed herein for reducing energy or food intake by administering to a subject a cosmetically or therapeutically effective amount of such an agonist. In one embodiment, administration of the receptor agonist results in a decrease in the amount, either the total weight or the total volume or calorie content of the food. In another embodiment, it may result in a decrease of the intake of a food component, such as a decrease in the ingestion of lipids, carbohydrates, cholesterol, or proteins. In any of the methods disclosed herein, the preferred compounds that have been discussed in details herein could be administered. In an additional embodiment, a method is disclosed herein for reducing appetite by administering a therapeutically effective amount of such an agonist. Appetite can be measured by any means known to one of skill in the art.

For example, decreased appetite can be assessed by a psychological assessment. In such an embodiment, administration of the receptor agonist results in a change in perceived hunger, satiety, and/or fullness. Hunger can be assessed by any means known to one of skill in the art. In one embodiment, hunger is assessed using psychological assays, such as by an assessment of hunger feelings and sensory perception using e.g. a questionnaire.

In a further embodiment, a method is disclosed herein for decreasing the motility of the upper GI tract as for example decreasing gastric emptying. PP, the prototype Y4 agonist is known to decrease gastric emptying. The method includes administering a therapeutically effective amount of such a Y4 selective agonist thereof to the subject, thereby decreasing GI-tract motility. It is well known that compounds which decrease gastric emptying will have a beneficial effect in also decreasing food intake as the subject is feeling more full or satiated.

In a further embodiment, a method is disclosed herein for altering energy metabolism in a subject. The method includes administering a therapeutically effective amount of such an agonist thereof to the subject, thereby altering energy expenditure. Energy is burned in all physiological processes. The body can alter the rate of energy expenditure directly, by modulating the efficiency of those processes, or changing the number and nature of processes that are occurring. For example, during digestion the body expends energy moving food through the bowel, and digesting food, and within cells, the efficiency of cellular metabolism can be altered to produce more or less heat. In a further embodiment a method is disclosed herein for any and all manipulations of the arcuate circuitry described in this application, which alter food intake coordinately and reciprocally alter energy expenditure. Energy expenditure is a result of cellular metabolism, protein synthesis, metabolic rate, and calorie utilization. Thus, in this embodiment, peripheral administration results in increased energy expenditure, and decreased efficiency of calorie utilization. In one embodiment, a therapeutically effective amount of a receptor agonist according to the invention is administered to a subject, thereby increasing energy expenditure.

In several embodiments both relating to the therapeutic use and to the cosmetic use, a Y4 selective agonist can be used for weight control and treatment, reduction or prevention of obesity, in particular any one or more of the following: preventing and reducing weight gain; inducing and promoting weight loss; and reducing obesity as measured by the Body Mass Index. As mentioned above, the invention also relates to the use of a Y4 selective agonist for controlling any one or more of appetite, satiety and hunger, in particular any one or more of the following: reducing, suppressing and inhibiting appetite; inducing, increasing, enhancing and promoting satiety and sensations of satiety; and reducing, inhibiting and suppressing hunger and sensations of hunger. The disclosure further relates to the use of a Y4 selective agonist in maintaining any one or more of a desired body weight, a desired Body Mass Index, a desired appearance and good health.

In a further or alternative aspect, the invention relates to a method for the treatment and/or prevention of reduced energy metabolism, feeding disorders, appetite disorders, overweight, obesity, bulimia, bulimia nervosa, Syndrome X (metabolic syndrome), or complications or risks associated thereto including diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, congestive heart failure, stroke, myocardial infarct, thromboembolic diseases, hypercholesterolemia, hyperlipidemia, gallbladder disease, osteoarthritis, sleep apnea, reproductive disorders such as polycystic ovarian syndrome, cancers of the breast, prostate, and colon, the method comprising administering to a subject such as a mammal including a human, an effective dose of one or more of a Y4 selective agonists as described herein.

### 2. Intestinal hypersecretion

PP is know to have a strong anti-secretory effect on both the small and large intestine and this appears to be mediated through Y4 receptors located on the epithelial cells (Cox & Tough 2001 Br.J.Pharmacol. 135: 1505-12). It has been shown in vivo that peripheral administration of PYY - another PP-fold peptide activating Y1 and Y2 receptors - can cause a long-lasting reduction in intestinal secretion induced by vasoactive intestinal polypeptide in human subjects with ileostomies (Playford et al 1990 Lancet 335: 1555-57). It was concluded that PYY could be a therapeutic agent against diarrhoea. However, for example the natriuretic and hypertensive effects of the combined Y1 and Y2 agonists, NPY and PYY peptides have prevented this. Such side effects will not be relevant for the selective Y4 receptor agonists of the present invention. Thus the selective Y4 agonists of the present invention are particularly useful for the treatment or protection against hyper-secretion of the GI-tract including various forms of diarrhoea whether or not they directly are caused by hyper-secretion as a suppression of the intestinal secretion will either eliminate the cause of the diarrhoea or eliminate the symptoms. One particularly interesting indication is the hyper-secretion observed in patients with ileostomia, who often are losing large amounts of fluid. The selective Y4 agonists of the present invention are particularly useful for the treatment or protection against hypersecretion associated with small intestinal ileo-stomia.

### 3. Emesis and Nausea

Many peptides and other types of compounds which have been suggested as agents to control appetite, such as for example PYY and CB1 antagonists are know to be emetic. For example, PYY was in fact discovered - for "the second time" - in 1989 as the biologically active entity in an intestinal extract causing dogs to vomit (Harding and McDonald 1989 Peptides 10: 21-24). It was concluded that PYY was the most potent, circulating emetic peptide identified and that this effect was mediated through area postreama known to have a leaky blood brain barrier. It has also been reported that PYY3-36 can cause nausea when administered peripherally to human subjects (Nastech press release 29^{th} of June 2004). Interestingly, it was noted that PP given in similar doses did not cause vomiting in these dogs (Harding and McDonald 1989). Thus, PP which acts through Y4 receptors also located in the area postreama of the brain stem - does not cause emesis or vomiting. Importantly, large doses of a combined Y2-Y4 agonist peptide - which has similar in vitro potency for the Y2 receptors as PYY - can be administered to animals such as cyno monkeys reaching very high plasma levels of 12-13.000 nM without observing any vomiting of the animals or evidence of GI-tract side effects This lack of emesis is surprising since PYY3-36 which has a similar potency on the Y2 receptor does cause emesis in man and conceivably in animals when administered at much lower doses. Thus, surprisingly the combined Y2-Y4 selective agonist does not cause emesis to the same degree as the selective Y2 agonist - PYY3-36 compound - does. Apparently, Y4 receptor activation - conceivably in the area postreama - prevents the emetic effect of the Y2 activation in this case an effect caused by the same compound acting on the Y2 receptors. Thus, the Y4 selective compounds of the present invention are particularly useful for the treatment or protection against emesis and nausea. This will be emesis and nausea associated with the treatment of for example another appetite suppressive agent for example of the Y2 agonist type, the CB1 antagonist /inverse agonist type or other types of appetite suppressive agents , which often cause emesis and nausea. It should be noted that the appetite suppressive effect of the Y4 selective compounds very likely will be additive or even synergistic at the same time. Thus, by co-administering a Y4 selective agonist of the present invention together with another appetite suppressive or other type of anti-obesity agent, two goals are achieved: 1) the beneficial effect of obtaining a fully or partial additive antiobesity effect, 2) the beneficial effect of the Y4 selective compound eliminating or diminishing the emetic effect of the other anti-obesity agent.

The Y4 selective compounds of the present invention, and PP itself, are also particularly useful for the treatment or protection against emesis and nausea associated with pregnancy. For this particular indication it is important that the Y4 selective compounds are close analogs of natural PP-fold peptides and generally are expected to have negligible side effects. Especially the fact that these peptides do not in the placenta cross from the maternal circulation into the fetal circulation is important since this will give minimal exposure of the fetus and thereby very low risk of causing developmental side effects.

The Y4 selective compounds of the present invention, and PP itself, are also useful for the treatment or protection against emesis and nausea associated with alcohol intolerance

### 4. Irritable bowl disease

The secretion and function of the natural ligand for the Y4 receptor, PP is highly correlated to the activity of the autonomous nervous system (Schwartz 1983, Gastroenterology 85:1411-25). Thus for example fluctuations in the plasma levels of PP are closely correlated to fluctuations in GI-tract motility and secretions and to secretion of the hormone/ neurotransmitter motilin. PP is known to work through activation of the parasympathetic nerveous system and through the central vagal control centers such as the vagal motor neurones in the brain stem and thereby controlling the activity in the efferent vagal fibers to the GI tract. Since irritable bowl disease is believed to be associated with malfunctions in especially the GI-tract motility and function leading to pain etc. and malfunction in the control of this through the autonomic nerveous system, the use of the selective Y4 agonists in the treatment of irritable bowl disease is a preferred embodiment of the present invention

### Additional comments concerning administration of Y4 agonists for the treatment or prevention of obesity and related diseases

During a meal a large repertoire of gastrointestinal hormones and neurotransmitter systems are activated in a carefully concerted, sequential and overlapping manner. Moreover, food components influence not only the secretion of GI hormones and the activity of various afferent neuronal pathways but these food components also influence various hormones and centers in the CNS directly after they are absorbed. Thus the regulation of food intake and energy expenditure is a highly complex and multifaceted process. In view of this it is surprising that certain hormones such as PP in fact can substantially affect the system when administered in a way which results in, for example only 3-4 times the plasma levels which are achieved during a meal.

Administrations of such compounds - Y4 selective agonists - apparently mainly have the intended effect if the compounds are given in the fasting state in an effective dose as described. If the Y4 agonists are given in a situation where the various hormonal and neuronal systems are active due to the presence of food components in the GI tract or the expectation of a meal, the effect is not seen or a smaller effect is observed. Thus, in a preferred embodiment of the invention the selective Y4 agonist is administered in the fasting state in an effective dose either sub-cutaneously, nasally or through other means as described elsewhere herein. In the present context, the term "fasted state" means that the subject has not eaten any food or drink within at least the last 2 hours before administration of the Y2 receptor agonist such as, e.g., within at least the last 3 hours, within at least the last 4 hours, within at least the last 5 hours, within at least the last 6 hours, within at least the last 7 hours, within at least the last 8 hours, within at least the last 9 hours, within at least the last 10 hours, within at least the last 11 hours or within at least the last 12 hours before dosing.

In a subgroup of the population, Y4 agonists may not have the intended action due to genetic variations such as polymorphisms in the Y4 receptor gene. Loss of function mutations in these receptors are likely to be associated with obesity. Thus, in a preferred embodiment of the invention an analysis of the Y4 gene of the subject to be treated is performed in order to probe for polymorphisms / mutations in these genes and identification of such polymorphisms. Based on such an analysis an optimal treatment of the subjects can be made. For example, only subjects with normal genotype or with polymorphisms, which do not affect the function of Y4 agonists, should be treated with such agonists. Another possibility is to increase the dose of the Y4 agonist in subjects who express an impaired receptor in order to ensure an optimal effect of the drug. In the case where the obesity of a subject is caused by an impairment in the function of the Y4 receptor it could be argued that treatment with a - for example large doses - of a Y4 agonist is a form of replacement therapy - provided that at least some of the relevant receptor function is still left - for example in heterozygote patients.

In one embodiment of the invention an acute test may be performed where a Y4 agonist is administered to ensure that these compounds have the intended effect in the subject to be treated before a chronic treatment is started. Through these means it is ensured that only subjects who are susceptible to treatment with Y4 agonists are treated with these compounds.

### Use of Y4 selective agonists in combination with Y2-selective agonists and other agents

Our copending International patent application filed on even date herewith discloses Y2-selective agonists and their use in treatment. The Appendix to this application briefly summarises the structural characteristics required of a Y2 agonist according to that application, and gives specific examples of Y2 selective agonists. As briefly discussed below, the Y4-agonists of this invention may be used in treatment together with Y2 specific agonists.

Through combined treatment with two compounds, respectively a selective Y4 agonist and a selectiveY2 agonist it is possible to dose each of these compounds freely i.e. independently of each other in order to obtain a maximal beneficial effect. That is, it is for example possible to administer a sub-maximal dose of the selective Y2 compound in order to perhaps ensure that only minimal side effects are obtained through for example peripheral cardiovascular Y2 receptors or generation of emesis, while at the same time administer a maximal or even supra-maximal dose of a Y4 agonist to ensure a very strong effect in all subjects through the Y4 receptor pathway, which has a broader therapeutic window. The concomitant - partial - stimulation of the Y2 receptor pathway will ensure a more efficient effect on appetite regulation and energy expenditure than the Y4 agonist stimulation alone. For example, the Y2 agonist may have a more pronounced effect on gastric emptying than the Y4 agonist. And, the concomitant stimulation of the Y4 receptor will allow for the use of a lower and safer dose of the Y2 agonist, which alone would not have been efficient.

In those cases where two individual compounds are used to obtain a combined stimulation of the Y4 and Y2 receptors, the administration regimen may be adjusted as mentioned above. Accordingly, in specific embodiments of the invention, a combination of a Y2 agonist and a Y4 agonist is administered so that the ratio between the dose of Y2 and the dose of Y4 is from about 0.1:10 to about 10:0.1 such as, e.g. from about 0.1:1 to about 1:0.1, from about 0.2:1 to about 1:0., from about 0.3:1 to about 1:0.3, from about 0.4:1 to about 1:0.4 or from about 0.5:1 to about 1:0.5 such as, e.g. 1:1, 1:2, 1:3, 1:4, 1:5, 1:6 etc. As mentioned above, an interesting embodiment is a combination of a Y2 and a Y4 agonist, wherein the balance between the amount of Y2 relative to Y4 is adjusted so that maximal effect is achieved with a minimum (if any) side effects. Accordingly, it is envisaged that the concentration of the Y2 agonist in such a combination is less than the concentration of the Y4 agonist. To this end, the concentration and the ratios mentioned above relates to the molar concentration and the molar ratio, respectively.

Thus in a preferred embodiment of the invention a selective Y4 agonist is administered in conjunction with a selective Y2 agonist for the treatment of obesity.

The compounds can be administered through the same administration route or through different administration routes. The administration route can in principle be any route such as those mentioned herein, the parenteral and the topical route being of most interest. Accordingly, in one embodiment of the invention the Y4 and Y2 agonists are both administered by e.g. the topical route such as by nasal administration or by subcutaneous injection and in another embodiment, the Y4 agonist is administered by nasal administration and the Y2 agonist is administered by subcutaneous injection or vice versa. Other suitable combinations or administration routes are within the scope of the present invention.

The Y4 and Y2 agonists used in a combination treatment may be contained in the same pharmaceutical composition (e.g. in admixture in a pharmaceutical composition) or they may be presented in separate pharmaceutical compositions for simultaneous, sequential or individualized administration with specific instructions for use. Accordingly, such compositions may be presented in the form of a kit comprising a Y4 agonist and a Y2 agonist in the same or in individual containers and, optionally with instructions for use.

Y4 specific agonists of the invention may be combined in the treatment of obesity, diabetes and related diseases with the use of various other drugs targeting appetite and energy expenditure, this includes but is not limited to drugs such as GI-tract lipase inhibitors, neurotransmitter reuptake inhibitors, cannabinoid receptors antagonists and inverse agonists, as well as other types of neurotransmitter - including but not limited to 5HT receptors - and/or hormone - including but not limited to GLP-1, MC4, MC3 - receptor agonist or antagonists. Due to the fact that the Y4 selective agonists are targeting a homeostatic regulatory mechanism in the communication between the GI-tract and the CNS - i.e. the Y4 receptors normally targeted by the satiety mediating hormone PP from the pancreas - it is particularly beneficial to combine the treatment with the Y4 selective agonist with the treatment with a drug targeting a central, hedonic mechanism in the regulation of appetite and energy expenditure, such as the CB1 receptors, for example being part of the reward system. Thus, the use of Y4 agonists in the treatment of obesity and related diseases in combination with a CB1 antagonist is a preferred embodiment of this invention.

### Dosages

The therapeutically effective amount of a Y4 receptor agonist according to the invention will be dependent on specific agonist employed, the age, weight and condition of subject being treated, the severity and type of the condition or disease being treated, the manner of administration and the strength of the composition applied.

For example, a therapeutically effective amount of a Y4 receptor agonist thereof can vary from about 0.01 µg per kilogram (kg) body weight to about 1 g per kg body weight, such as about 1 µg to about 5 mg per kg body weight, or about 5 µg to about 1 mg per kg body weight. In another embodiment, the receptor agonist is administered to a subject at 0.5 to 135 picomole (pmol) per kg body weight, or about 72 pmol per kg body weight.

In one specific, non-limiting example from about 5 to about 50 nmol is administered as a subcutaneous injection, such as from about 2 to about 20 nmol, or about 1.0 nmol is administered as a subcutaneous injection. The exact dose is readily determined by one skilled in the art based on the potency of the specific compound (such as the receptor agonist) utilized, the age, weight, sex and physiological condition of the subject. The dose of an agonist can be a molar equivalent of the therapeutically effective dose of PYY3-36.

The amounts can be divided into one or several doses for administration daily, every second day, weekly, every two weeks, monthly or with any other suitable frequency. Normally, the administration is once or twice daily.

### Methods of administration

The Y4 receptor agonist as well as cosmetic or pharmaceutical compositions according to the invention can be administered by any route, including the enteral (e.g. oral administration) or parenteral route. In a specific embodiment, the parenteral route is preferred and includes intravenous, intraarticular, intraperitoneal, subcutaneous, intramuscular, intrasternal injection and infusion as well as administration by the sublingual, transdermal, topical, transmucosal including nasal route, or by inhalation such as, e.g., pulmonary inhalation. In specific embodiments, the subcutaneous and/or the nasal administration route is preferred.

When administered centrally, the natural Y4 selective peptide PP may, as do NPY and PYY when administered ICV, induce eating (probably dye to activation of central receptors which normally are not reached by the circulating hormones or peripherally administered peptide compounds). Thus in cases where increased eating is to be avoided, it is preferred that the Y4 selective agonists of the invention are administered peripherally.

The receptor agonists can be administered as such dispersed in a suitable vehicle or they can be administered in the form of a suitable pharmaceutical or cosmetic composition. Such compositions are also within the scope of the invention. In the following are described suitable pharmaceutical compositions. A person skilled in the art will know how that such composition may also be suitable for cosmetic use or he will know how to adjust the compositions to cosmetic compositions by use of suitable cosmetically acceptable excipients.

### Pharmaceutical compositions

The receptor agonists (also denoted "compounds") according to the invention for use in medicine or cosmetics are normally presented in the form of a pharmaceutical composition comprising the specific compound or a derivative thereof together with one or more physiologically or pharmaceutically acceptable excipients.

The compounds may be administered to an animal including a mammal such as, e.g., a human by any convenient administration route such as, e.g., the oral, buccal, nasal, ocular, pulmonary, topical, transdermal, vaginal, rectal, ocular, parenteral (including inter alia subcutaneous, intramuscular, and intravenous cf. above), route in a dose that is effective for the individual purposes. A person skilled in the art will know how to chose a suitable administration route. As mentioned above, the parenteral administration route is preferred. In a specific embodiment, the receptor agonists are administered subcutaneously and/or nasally. It is well known in the art that subcutaneous injections can be easily self-administered.

A composition suitable for a specific administration route is easily determined by a medical practitioner for each patient individually. Various pharmaceutically acceptable carriers and their formulation are described in standard formulation treatises, e.g., Remington's Pharmaceutical Sciences by E. W. Martin.

The pharmaceutical composition comprising a compound according to the invention may be in the form of a solid, semi-solid or fluid composition. For parenteral use, the composition is normally in the form of a fluid composition or in the form of a semi-solid or solid form for implantation.

Fluid compositions, which are sterile solutions or dispersions can utilized by for example intravenous, intramuscular, intrathecal, epidural, intraperitoneal or subcutaneous injection of infusion. The compounds may also be prepared as a sterile solid composition, which may be dissolved or dispersed before or at the time of administration using e.g. sterile water, saline or other appropriate sterile injectable medium.

The fluid form of the composition may be a solution, an emulsion including nano-emulsions, a suspension, a dispersion, a liposomal composition, a mixture, a spray, or a aerosol (the two latter types are especially relevant for nasal administration).

Suitable mediums for solutions or dispersions are normally based on water or pharmaceutically acceptable solvents e.g. like an oil (e.g. sesame or peanut oil) or an organic solvent like e.g. propanol or isopropanol. A composition according to the invention may comprise further pharmaceutically acceptable excipients such as, e.g., pH adjusting agents, osmotically active agents e.g. in order to adjust the isotonicity of the composition to physiologically acceptable levels, viscosity adjusting agents, suspending agents, emulsifiers, stabilizers, preservatives, antioxidants etc. A preferred medium is water.

Compositions for nasal administration may also contain suitable non-irritating vehicles such as, e.g., polyethylene glycols, glycofurol, etc. as well as absorption enhancers well known by a person skilled in the art (e.g. with reference to Remington's Pharmaceutical Science)

For parenteral administration, in one embodiment the receptor agonists can be formulated generally by mixing it at the desired degree of purity, in a unit dosage injectable form (solution, suspension, or emulsion), with a pharmaceutically acceptable excipient or carrier, i.e., one that is non-toxic to recipients at the dosages and concentrations employed and is compatible with other ingredients of the composition.

Generally, the formulations are prepared by contacting the receptor agonist uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes. Due to the amphiphatic nature of the peptides described herein suitable forms also include micellar formulations, liposomes and other types of formulations comprising one or more suitable lipids such as, e.g., phospholipids and the like.

Preferably, they are suspended in an aqueous carrier, for example, in an isotonic buffer solution at a pH of about 3.0 to about 8.0, preferably at a pH of about 3.5 to about 7.4, 3.5 to 6.0, or 3.5 to about 5. Useful buffer substances include acetate, citrate, phosphate, borate, carbonate such as, e.g., sodium citrate-citric acid and sodium phosphate-phosphoric acid, and sodium acetate/acetic acid buffers.

The compositions may also be designed to controlled or prolonged delivery of the receptor agonist after administration in order to obtain a less frequent administration regimen. Normally a dosage regimen including 1-2 daily administrations is considered suitable, but within the scope of the present invention is also included other administration regimens such as, e.g., more frequent and less frequent. In order to achieve a prolonged delivery of the receptor agonist, a suitable vehicle including e.g. lipids or oils may be employed in order to form a depot at the administration site from which the receptor agonist is slowly released into the circulatory system, or an implant may be used. Suitable compositions in this respect include liposomes and biodegradable particles into which the receptor agonist has been incorporated.

In those situations where solid compositions are required, the solid composition may be in the form of tablets such as, e.g. conventional tablets, effervescent tablets, coated tablets, melt tablets or sublingual tablets, pellets, powders, granules, granulates, particulate material, solid dispersions or solid solutions.

A semi-solid form of the composition may be a chewing gum, an ointment, a cream, a liniment, a paste, a gel or a hydrogel.

Other suitable dosages forms of the pharmaceutical compositions according to the invention may be vagitories, suppositories, plasters, patches, tablets, capsules, sachets, troches, devices etc.

The dosage form may be designed to release the compound freely or in a controlled manner e.g. with respect to tablets by suitable coatings.

The pharmaceutical composition may comprise a therapeutically effective amount of a compound according to the invention.

The content of a compound of the invention in a pharmaceutical composition of the invention is e.g. from about 0.1 to about 100% w/w of the pharmaceutical composition.

The pharmaceutical compositions may be prepared by any of the method well known to a person skilled in pharmaceutical formulation.

In pharmaceutical compositions, the compounds are normally combined with a pharmaceutical excipient, i.e. a therapeutically inert substance or carrier.

The carrier may take a wide variety of forms depending on the desired dosage form and administration route.

The pharmaceutically acceptable excipients may be e.g. fillers, binders, disintegrants, diluents, glidants, solvents, emulsifying agents, suspending agents, stabilizers, enhancers, flavours, colors, pH adjusting agents, retarding agents, wetting agents, surface active agents, preservatives, antioxidants etc. Details can be found in pharmaceutical handbooks such as, e.g., Remington's Pharmaceutical Science or Pharmaceutical Excipient Handbook.

The following examples describe the preparation and activities of some specific agonists of the invention.

### Syntheses

Peptidic agonists of the invention may be synthesized by solid phase peptide synthesis, using either an automated peptide synthesizer, or traditional bench synthesis. The solid support can be, for example, chlorotrityl (CI) or Wang (OH) resin, both of which are readily available commercially. The active groups of those resins react readily with the carboxyl group of an N-Fmoc amino acid, thereby covalently binding it to the polymer. The resin-bound amine may be deprotected by exposure to piperidine. A second N-protected amino acid may then be coupled to the resin-amino acid. These steps are repeated until the desired sequence is obtained. At the end of the synthesis, the resin-bound protected peptide may be deprotected and cleaved from the resin with trifluoroacetic acid (TFA). Examples of reagents facilitating the coupling new amino acids to the resin-bound amino acid chain are: tetra-methyluronium hexafluorophosphate (HATU), O-(1H-benzotriazole-1-yl)-N,N,N',N'-tetramethyluronium hexafluorophosphate (HBTU), O-(1H-benzotriazole-1-yl)-N,N,N',N'-tetramethyluronium tetrafluoroborate (TBTU), 1 H-hydroxybenzotriazole (HOBt).

Peptide synthesis by solution chemistry rather than solid phase chemistry is also feasible.
Modification of a side-chain amino or carboxyl group of an amino acid in the peptide chain, for example to introduce a GAG-binding or other motif as described above, is a simple matter of selective protection and deprotection of other reactive side-chain groups not to be involved in the reaction.

The peptides referred to herein are made by solid phase synthesis, on PAL Peg-PS resin, (amide resin (amide resin Applied Bioscience, Warrington, UK GEN913401), using Fmoc chemistry with a 5x reagent excess. The coupling was performed by HCTU throughout, solvent DMF. Fmoc removal was performed with 20% piperidine in DMF, 10-15 minutes. However, these peptides could just as well have been synthesised by various other standard peptide synthesis methods such as tBOC chemistry and solution chemistry instead of solid state etc. The synthesis is illustrated by the following description, but other peptides with which the invention is concerned were made by similar methods:

### Synthesis of [Cys2, D-Cys27]PP

In general side group protection were standard Fmoc except for:
- Arg: = Fmoc Arg(Pbf) -OH
- Asn, Gln: = Fmoc Asn(Trt)-OH
- Thr, Ser, Asp, Glu, Tyr: = tButyl
- Ala-Ser 22-23: = Fmoc AlaSer pseudoproline

In the case of [Cys2, D-Cys27]PP the following special protection groups were used in order to obtain selective deprotection:
- Cys 27: = Fmoc DLys(Trt) -OH

The peptide was synthesized by solid phase synthesis, on PAL Peg-PS resin (a resin which will generate the biologically important carboxyamide group upon cleavage), using Fmoc chemistry with a 5 fold molar reagent excess. The coupling was performed by HCTU throughout using DMF as solvent. Fmoc removal after each coupling step was performed with 20% piperidine in DMF for 10-15 minutes. The coupling was checked after each step by quantitative ninhydrin assay. In certain cases double couplings could be performed.

The resin was subsequently divided into different batches to generate the peptide.

As further illustration of the synthetic methods which may be used for the preparation of the Y4 selective agonists with which the invention is concerned, the following protocols are set out, by way of example only:

### Synthesis of [Lys13]PP2-36 (SEQ ID No: 54) and analogs thereof

In the following, a synthesis of the Y4 selective peptide [Lys13]PP2-36 and the synthesis of analogs with either a GAG-binding motif, a serum protein binding motif or a polyethyleneglycol moiety attached at the epsilon amino group of Lys13, is described.

In general side group protection is standard Fmoc except for:
- Arg: = Fmoc Arg(Pbf) -OH
- Asn, Gln: = Fmoc Asn(Trt)-OH
- Thr, Ser, Asp, Glu, Tyr: = tButyl
- Ala-Ser 22-23: = Fmoc AlaSer pseudoproline

In the case of [Lys13]PP2-36 the following special protection groups were used in order to obtain selective deprotection:
- Lys 13: = Fmoc Lys(Dde)- OH

The peptide is synthesized by solid phase synthesis, on PAL Peg-PS resin (a resin which will generate the biologically important carboxyamide group upon cleavage), using Fmoc chemistry with a 5 fold molar reagent excess. The coupling is performed by HCTU throughout using DMF as solvent. Fmoc removal after each coupling step is performed with 20% piperidine in DMF for 10-15 minutes. The coupling is checked after each step by quantitative ninhydrin assay. In certain cases double couplings could be performed.

After the synthesis of the full length peptide, the protection group on the epsilon amino group of Lys13 is selectively removed by treatment with 2 % hydrazine in DMF for 15-20mins, while the peptide is still attached to the resin.

The resin is subsequently divided into different batches to generate the underivatized peptide as well as three different motif-modified peptides:
1. [Lys13]PP2-36- the "mother peptide" - is cleaved from the resin and deprotected by treatment with 95% trifluoroacetic acid : 2.5% water: 2.5% tripropyl silane for 2-3 hours
   The peptide is purified by reverse phase HPLC: Vydac 300A column, 250mm x 10mm column eluted with (Buffer A = 0.05% TFA in water; Buffer B = 60%MeCN: 40% water: 0.05% TFA) in a gradient of 30-80% buffer B over 20mins, 2ml/min. OD 215 nM was measured and the eluant containing the specific peptide is collected and lyophilized.
   The structure of the peptide is confirmed by mass spec, amino acid analysis and if desired also by amino acid sequence analysis.
**2. [N-(8-(8-gammaglutamoylamino-octanoylamino)-octanoyl)- [Lys13]PP2-36 (SEQ ID No: 55)** -In order to attach a serum albumin binding motif, an 8-(8-gammaglutamoylamino-octanoylamino)-octanoyl group is linked to the free epsilon amino group of Lys13 after removal of the protecting Dde group while the peptide is still attached to the resin by peptide synthesis using protected aminooctanoic acid twice followed by protected gammaglutamic acid.
   The motif-modified peptide is cleaved from the resin, deprotected, and purified as described above for the "mother peptides".
**3. Fluorescein-[N-{(Ala-Arg-Arg-Arg-Ala-Ala-Arg-Ala)₃}-[Lys13]PP2-36 (SEQ ID No: 39) -** In order to attach a GAG-binding motif, the motif is built stepwise by peptide synthesis using the free epsilon amino group of Lys13, after removal of the Dde group, on the peptide still attached to the resin, using standard Fmoc chemistry as described above in general: The GAG binding sequence attached in this way is Ala-Arg-Arg-Arg-Ala-Ala-Arg-Ala-Ala-Arg-Arg-Arg-Ala-Ala-Arg-Ala-Ala-Arg-Arg-Arg-Ala-Ala-Arg-Ala. A fluorescein tag group is added for in vitro and in vivo analytical purposes as its 5,6 isomer - NHS ester, 10x excess over 72 hours - to the N-terminus of the final GAG-binding sequence.
   The GAG binding-motif modified peptide is cleaved from the resin, deprotected, and purified as described above for the "mother peptide".
**4. [N-{N'-(21-amino-4,7,10,13,16,19-hexaoxaheneicosanoyl)}-gammaglutamoyl-Lys13]PP2-36 (SEQ ID No: 40)**
   - In order to attach a polyethylenglycol moiety to the Y4 selective peptide, protected 21-amino-4,7,10,13,16,19-hexaoxaheneicosanoic-acid is joined by peptide synthesis using the free epsilon amino group of Lys13 after removal of the Dde grup on the peptide still attached to the resin, followed by a protected gamma-glutamic acid.

The PEGylated peptide is cleaved from the resin, deprotected, and purified as described above for the "mother peptide".

| ***Structure*** | **Molecular formula** | **Mw** | **Theoretical m/z** | **Measured Mass m/z** | **Rt min** | **Purity %** | **Analytical method** |
|---|---|---|---|---|---|---|---|
| PP2-36 | C182H282N5 | 4110.7 | | 4109.2 | 18.1 | 96.2 | C |
| | 2053S2 | | | | | | |
| [His34]-PP | C186H286N5 4055S2 | 4222.1 | 704.7 [M+6H] | 704.6 [M+6H} | 14.7 | 95.6 | B |
| [Pro34]-PYY | C195 H294 N54 057 | 4306.7 | | 4279.6 [M+H] | 13.5 | 91.6 | A |
| [Ala1,Pro34]-PYY | C189 H290 N54 056 | 4214.7 | | 4188.3 [M+H] | 13.1 | 95.0 | A |
| [Ala30,Pro34]-PYY | C192 H288 N54 057 | 4264.7 | | 4239.0 [M+H] | 13.7 | 90.0 | A |
| [Glu4,Pro34]PYY | C194 H290 N54 058 | 4306.7 | | 4281.8 | 13.2 | 100 | A |
| [Arg26,Pro34]PYY | C194 H298 N54 057 | 4298.8 | | 4300.1 | 13.4 | 98.9 | A |
| [Ile28,Pro34]PYY | C195 H294 N54 057 | 4306.8 | | 4281.2 | 13.6 | 97.0 | A |
| Met30,Pro34]PYY | C194H292N5 4 O57 S | 4324.8 | | 4298.5 | 13.2 | 96.9 | A |
| [Cys2,D-Cys27]-PP | C177H279N5 3053S4 | 4125.8 | | 4124.4 | 17.9 | 98.7 | C |
| [CLTRPRY-amide]x2 (SEQ ID No: 18) | C80H130N28 O20 S2 | 1868.2 | | 1812.1 | 10.0 | 99.3 | A |
| [CTRPRY-amide]x2 (SEQ ID No: 7) | C68H108N26 O18 S2 | 1641.9 | | 1584.9 | 8.9 | 100 | A |

### Analytical HPLC method A

| | |
|---|---|
| Column | = Vydac C18 Peptide-Protein column, 250 x 4.6 mm |
| Buffer A | = 0.05% TFA in water |
| Buffer B | = 0.05% TFA in 100% MeCN |
| Gradient | = 0% B to 60% B in 20 min |
| Flow rate | = 1.00 mL/min |
| Wavelength | = 215 nm |

Mass spectroscopy = MALDI-TOF with gentisic acid or □cyanohydroxy cinnamic acid as matrix.

### Analytical HPLC method B

| | |
|---|---|
| Column | = Hypersil ODS-2, 250x4.6 mm |
| Buffer A | = 0.1 % TFA in 100% MeCN |
| Buffer B | = 0.1 % TFA in 100% water |
| Gradient | = 24% A to 35% A in 25 min |
| Flow rate | = 1.00 mL/min |
| Wavelength | = 220 nm |

Mass spectroscopy = ESI [nebuliser gas flow: 1.5 L/min; CDL -20.0v; CDL temp: 250°C; Block temp: 200°C; probe bias: +4.5kv; Detector: 1.5 kv; T. Flow: 0.2 mL/min; B. conc: 50% H20/50% CAN.]

### Analytical HPLC method C

| | |
|---|---|
| Column | = Vydac C18 218TP54, 250 x 4.6 mm |
| Buffer A | = 20 mL of MeCN and 2 mL of TFA in Water (total volume 2000 mL) |
| Buffer B | = 2 mL of TFA in Water (total volume 2000 mL) |
| Gradient | = 25% B to 75% B over 27 min |
| Flow rate | = 1.00 mL/min |
| Wavelength | = 215 nm |
| | |
| Injection volume | = 10 µL |

### Biological Assays and Results

### I. IN VITRO ASSAYS TO DETERMINE PEPTIDE AFFINITY AND POTENCY

### Human Y2 receptor Affinity Assay

Affinity of test compounds for the human Y2 receptor is determined in a competition binding assay using 1251-PYY binding in COS-7 cells transiently transfected with the human Y2 receptor using a standard calcium phosphate transfection method.

Transfected COS-7 cells are transferred to culture plates one day after transfection at a density of 40 x 103 cells per well aiming at 5 - 8 % binding of the radioactive ligand. Two days after transfection, competition binding experiments are performed for 3 hours at 4 C° using 25 pM of 1251-PYY (Amersham, Little Chalfont, UK). Binding assays are performed in 0.5 ml of a 50 mM Hepes buffer, pH 7.4, supplemented with 1 mM CaCl2, 5 mM MgCl2, and 0.1 % (w/v) bovine serum albumin and 100 µg/ml bacitracin. Non-specific binding is determined as the binding in the presence of 1 µM of unlabeled PYY. Cells are washed twice in 0.5 ml of ice-cold buffer and 0.5-1 ml of lysis buffer (8 M Urea, 2 % NP40 in 3 M acetic acid) is added and the bound radioactivity is counted in a gamma counter. Determinations are made in duplicate. Steady state binding is reached with the radioactive ligand under these conditions.

EC50 values were calculated using a standard pharmacological data handling software, Prism 3.0 (graphPad Sofware, San Diego, USA).

### Human Y4 receptor Affinity Assay

Protocol as for the Y2 affinity assay, except that human Y4-transformed COS-7 cells are used, the competition assay uses 125I-PP, and PP is used for the determination of non-specific binding.

### Human Y1 receptor Affinity Assay

Protocol as for the Y2 affinity assay, except that human Y1-transformed COS-7 cells are used and are transferred to culture plates at a density of 1.5 x 106 cells per well. the competition assay uses 125I-NPY, and NPY is used for the determination of non-specific binding.

### Human Y5 receptor Affinity Assay

Protocol as for the Y2 affinity assay, except that human Y5-transformed COS-7 cells are used and are transferred to culture plates at a density of 5 x 105 cells per well. the competition assay uses 125I-NPY, and NPY is used for the determination of non-specific binding.

The results of testing NPY, PYY. PYY3-36, PP and three agonists of the invention in the above affinity assays are given in Table 1:

**Table 1**

| Agonist | Y2 | | | Y1 | | | Y4 | | |
|---|---|---|---|---|---|---|---|---|---|
| | IC50 Nm | SEM | n | IC50 nm | SEM | n | IC50 nm | SEM | n |
| NPY | 0.30 | 0.07 | 4 | 2.3 | 0.3 | 4 | 26 | 5 | 4 |
| PYY | 0.22 | 0.02 | 2 | 16 | 1 | 2 | 30 | 8 | 2 |
| PYY(3-36) | 0.20 | 0.03 | 1 0 | >1000 | | 2 | 343 | 42 | 4 |
| PP | >1000 | | 4 | >1000 | | 1 | 0.41 | 0.05 | 11 |
| | | | | | | | | | |
| PP2-26 | >1000 | | 3 | >1000 | | 2 | 0.64 | 0.04 | 4 |
| [Cys2,D-Cys27]PP | >1000 | | 3 | >1000 | | 2 | 8.2 | 1.1 | 4 |
| [Cys2,Aoc5-24,D-Cys27]PP | >1000 | | 4 | >1000 | | 2 | 286 | 30 | 4 |
| [His34]PP | >1000 | | 2 | >1000 | | 2 | 0.48 | 0.03 | 2 |
| [Ala1,Pro34]PYY | >1000 | | 2 | 176 | | 1 | 4.4 | 1.6 | 2 |
| [Glu4,Pro34]PYY | >1000 | | 2 | 99 | 23 | 2 | 2.4 | 1.2 | 2 |
| [Arg26,Pro34]PYY | 177 | 9 | 2 | 15 | | 1 | 0.43 | 0.09 | 2 |
| [Met30,Pro34]PYY | 969 | 18 | 2 | 88 | 27 | 2 | 2.3 | 1.0 | 2 |
| [Ala30,Pro34]PYY | >1000 | | 1 | 173 | | 1 | 0.99 | | 1 |
| [CTRPRY- | >1000 | | 1 | >1000 | | 1 | 29 | | 1 |
| amide]x2 | | | | | | | | | |
| [CLTRPRY-amide]x2 | >1000 | | 1 | >1000 | | 1 | 1.3 | | 1 |

### Human Y2 receptor Potency Assay

Potency of the test compounds on the human Y2 receptor is determined by performing dose-response experiments in COS-7 cells transiently transfected with the human Y2 receptor as well as a promiscuous G protein, Gqi5 which ensures that the Y2 receptor couples through a Gq pathway leading to an increase in inositol phosphate turnover.

*Phosphatidylinositol turnover* - One day after transfection COS-7 cells are incubated for 24 hours with 5 µCi of [3H]-myo-inositol (Amersham, PT6-271) in 1 ml medium supplemented with 10% fetal calf serum, 2 mM glutamine and 0.01 mg/ml gentamicin per well. Cells are washed twice in buffer, 20 mM HEPES, pH 7.4, supplemented with 140 mM NaCl, 5 mM KCI, 1 mM MgSO4, 1 mM CaCl2, 10 mM glucose, 0.05 % (w/v) bovine serum; and are incubated in 0.5 ml buffer supplemented with 10 mM LiCl at 37C for 30 min. After stimulation with various concentrations of peptide for 45 min at 37C, cells are extracted with 10 % ice-cold perchloric acid followed by incubation on ice for 30 min. The resulting supernatants are neutralized with KOH in HEPES buffer, and the generated [3H]-inositol phosphate are purified on Bio-Rad AG 1-X8 anion-exchange resin and counted in a beta counter. Determinations are made in duplicates. EC50 values were calculated using a standard pharmacological data handling software, Prism 3.0 (graphPad Sofware, San Diego, USA).

### Human Y4 receptor Potency Assay

Protocol as for the Y2 potency assay, except that human Y4-transformed COS-7 cells are used.

### Human Y1 receptor Potency Assay

Protocol as for the Y2 potency assay, except that human Y1-transformed COS-7 cells are used.

### Human Y5 receptor Potency Assay

Protocol as for the Y2 potency assay, except that human Y5-transformed COS-7 cells are used

The results of testing NPY, PYY, PYY3-36, PP and four of the agonists of the invention in the above potency assays are given in Table 2:

**Table 2**

| Agonist | Y2 | | | Y1 | | | Y4 | | |
|---|---|---|---|---|---|---|---|---|---|
| | EC50 nm | SEM | n | EC50 nm | SEM | n | EC50 nm | SEM | n |
| NPY | 1.5 | 0.5 | 11 | 1.7 | 0.4 | 11 | 167 | 56 | 7 |
| PYY | 0.23 | 0.06 | 8 | 0.6 | 0.1 | 5 | 34 | 5 | 6 |
| PYY(3-36) | 0.36 | 0.07 | 16 | 74 | 5 | 7 | >1000 | | 8 |
| PP | >1000 | | 8 | 83 | 13 | 5 | 0.64 | 0.07 | 17 |
| | | | | | | | | | |
| PP 2-36 | >1000 | | 4 | 297 | 45 | 3 | 0.88 | 0.12 | 5 |
| [Cys2,D-Cys27]-PP | >1000 | | 5 | >1000 | | 3 | 4.6 | 0.6 | 5 |
| [His34]-PP | >1000 | | 4 | >1000 | | 4 | 1.2 | 0.0 | 4 |

### II. IN VITRO ASSAYS TO DETERMINE PROTEIN STABILITY

An important measure for many of the peptides of the invention is the protein stability especially in respect of stability for example towards degradation by enzymes as the peptides have been designed to have increased stability as compared to for example PYY3-36 or even increased stability as compared to full length PYY and PP.

**Stability of the PP-fold** - is determined as the stability of the peptides towards degradation by endopeptidases which cleave for example in the loop region, which is relatively flexible as described (*O'Hare*, *M. & Schwartz, T.W*. *1990 In Degradation of Bioactive Substances: Physiology and Pathophysiology. J.Henriksen, ed. CRC Press, Boca Raton, FI*.). As a model enzyme, endoproteinase Asp-N (Pierce) is used. This enzyme cleave at the N-terminal side of Asp residues, for example between residues 9 and 10 (Asp) in PP. The peptides are incubated with an efficacious dose of endopeptidase Asp-N as indicated by the manufacturer in 0.01 M Tris/HCl buffer, pH 7.5 at room temperature and samples removed after various time periods over 24 hours. The samples are analysed by HPLC and the gradual degradation of the peptides is followed over time. The peptides are compared in respect of stability to PYY, PYY13-36 and PP.

**Stability towards aminopeptidases** - is determined as described above for endopeptidases but using aminopeptidase N and di-peptidylpeptidase IV in stead. Some of the peptides are specially designed to be resistant to these aminopeptidase, for example PYY2-36, the N-terminally acetylated peptide derivates, and peptide derivates alkylated with an albumin binding moiety at the N-terminus. The peptides are compared in respect of stability to PYY, PYY3-36 and PP.

### III. IN VITRO ASSAY TO DETERMINE BINDING TO GLYCOSAMINO GLYCANS (GAGS)

The ability of test compounds to bind to GAGs is monitored in an in vitro assay using immobilized heparin, i.e. a heparin agarose as affinity matrix. using either HiTrap heparin-Sepharose column (Amersham Pharmacia Biotech, Uppsala, Sweden) or heparin HPLC columns which are eluted with a 50-min linear gradient of 0-0.5 M NaCl in 50 mM sodium phosphate (pH 7.3) containing 2 mM DTT and 1 mM MgEDTA at a flow rate of 1 ml/min. For regeneration, the column was washed with 1 M NaCl in buffer A from 51-55 min.

### IV. IN VIVO STUDIES TO DETERMINE THE EFFECT OF THE PEPTIDES ON APPETITE, FOOD INTAKE AND BODY WEIGHT

Effect of Y4 selective agonists on acute food intake in mice
The ddy strain of mice were used, 34-37 g and 8-9 weeks of age (Japan SLC, Shizuuoka, Japan). The mice were individually house in a regulated environment (22 C, 55 % humidity) in a 12-hour light-dark cycle with light on at 7 AM. Food and water were available ad libitum except just before experiments (see below). The mice were acclimatized to subcutaneous injections during the week before experiments started. Mice were used once each in the experiments. Just before administration the peptides were diluted in physiological saline and administered in 100 µL volume for subcutaneous administration. Results are expressed as mean +/- SE. Analysis of variance followed by Bonferroni's test were used to assess differences among groups.

Mice were deprived of food but with free access to water for 16 hours prior to the actual test and experiments were started at 10 AM on the following day. A standard diet was used (CLEA Japan, Inc., Tokyo, Japan) and food intake was measured by subtracting uneaten food from the initially premeausured food after administration and checking for food spillage. Eight animals in each group receiving either saline, 1 µg PP2-36, or 10 µg PP2-36 test compound.

The results for PP2-36 (Seq ID No: 10) as test compound are shown in Fig. 2.

SEQUENCES:

NPY (SEQ ID No: 1)
H2N-Tyr--Pro-Ser-Lys-Pro-Asp-Asn-Pro-Gly-Glu-Asp-Ala-Pro-Ala-Glu-Asp-Met-Ala-
Arg-Tyr-Tyr-Ser-Ala-Leu-Arg-His-Tyr-Ile-Asn-Leu-Ile-Thr-Arg-Gln-Arg-Tyr-CONH2

PYY (SEQ ID No: 2)
H2N-Tyr-Pro-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Leu-Val-Thr-Arg-Gln-Arg-Tyr-CONH2

PP (SEQ ID NO: 3)
H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Met-Leu-Thr-Arg-Pro-Arg-Tyr-CONH2

[Cys2,DCys27]-PP SEQ ID. No:4)
S
|
H2N-Ala-Cys-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-(D-Cys)-Ile-Asn-Met-Leu-Thr-Arg-Pro-Arg-Tyr-
CONH2
|
S

[Lys28,Glu32]PP25-36 (SEQ ID. No:5)
H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Lys-Asn-Met-Leu-Glu-Arg-Pro-Arg-Tyr-CONH2

[Glu28,Lys32]PP25-36 (SEQ ID. No:6).
H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Glu-Asn-Met-Leu-Lys-Arg-Pro-Arg-Tyr-CONH2

S-Cys-Thr-Arg-Pro-Arg-Tyr-CONH2
|
S-Cys-Thr-Arg-Pro-Arg-Tyr-CONH2 (SEQ ID. No:7)

S-Cys-Thr-Arg-Pro-Arg-Tyr-CONH2
|
S-Cys-Thr-Arg-His-Arg-Tyr-CONH2 (SEQ ID. No:8)

[Cys2,Aoc5-24,Dcys27]-PP (SEQ ID. No:9).
S ———————————— S
|                                    |
H2N-Ala-Cys-Leu-Glu-Aoc5-24-Arg-Arg-(D-Cys)--Ile-Asn-Met-Leu-Thr-Arg-Pro-Arg-
Tyr-CONH2

PP2-36 (SEQ ID. No:10).
H2N-Pro-Leu-Glu-Pro-Val-Tyr-Pro-Gly-Asp-Asn-Ala-Thr-Pro-Glu-Gln-Met-Ala-Gln-
Tyr-Ala-Ala-Asp-Leu-Arg-Arg-Tyr-Ile-Asn-Met-Leu-Thr-Arg-Pro-Arg-Tyr-CONH2

[His34]-PP (SEQ ID. No:11).
H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Met-Leu-Thr-Arg-His-Arg-Tyr-CONH2

[Ala1,Pro34]-PYY (SEQ ID. No:12).
H2N-Ala-Pro-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Leu-Val-Thr-Arg-Pro-Arg-Tyr-CONH2

[Ala2,Pro34]-PYY (SEQ ID. No:13).
H2N-Tyr-Ala-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Leu-Val-Thr-Arg-Pro-Arg-Tyr-CONH2

[Glu4,Pro34]-PYY (SEQ ID. No:14).
H2N-Tyr-Pro-Ile-Glu-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Leu-Val-Thr-Arg-Pro-Arg-Tyr-CONH2

[Arg26,Pro34]-PYY (SEQ ID. No:15).
H2N-Tyr-Pro-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-Arg-Tyr-Leu-Asn-Leu-Val-Thr-Arg-Pro-Arg-Tyr-CONH2

[Ile28,Pro34]-PYY (SEQ ID. No:16).
H2N-Tyr-Pro-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Ile-Asn-Leu-Val-Thr-Arg-Pro-Arg-Tyr-CONH2

[Met30,Pro34]-PYY (SEQ ID. No:17).
H2N-Tyr-Pro-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Met-Val-Thr-Arg-Pro-Arg-Tyr-CONH2

S-Cys-Leu-Thr-Arg-Pro-Arg-Tyr-CONH2
|
S-Cys-Leu-Thr-Arg-Pro-Arg-Tyr-CONH2 (SEQ ID. No:18).

S-Cys-Leu-Thr-Arg-His-Arg-Tyr-CONH2
|
S-Cys-Leu-Thr-Arg-His-Arg-Tyr-CONH2 (SEQ ID. No:19).

[tetradecanoyl-Ala1]-PP (SEQ ID No:20)

CH2(CH2)12CONH-Ala-Pro-Leu-Glu-Pro-Val-Tyr-Pro-Gly-Asp-Asn-Ala-Thr-Pro-Glu-
Gln-Met-Ala-Gln-Tyr-Ala-Ala-Asp-Leu-Arg-Arg-Tyr-Ile-Asn-Met-Leu-Thr-Arg-Pro-Arg-
Tyr-CONH2

[tetradecanoyl-Ala1,His34]-PP (SEQ ID No:21)

CH2(CH2)12CONH-Ala-Pro-Leu-Glu-Pro-Val-Tyr-Pro-Gly-Asp-Asn-Ala-Thr-Pro-Glu-
Gln-Met-Ala-Gln-Tyr-Ala-Ala-Asp-Leu-Arg-Arg-Tyr-Ile-Asn-Met-Leu-Thr-Arg-His-Arg-
Tyr-CONH2

[Lys18,His34]-PP (SEQ ID No:22)

H2N-Ala-Pro-Leu-Glu-Pro-Val-Tyr-Pro-Gly-Asp-Asn-Ala-Thr-Pro-Glu-Gln-Met-Lys-
Gln-Tyr-Ala-Ala-Asp-Leu-Arg-Arg-Tyr-Ile-Asn-Met-Leu-Thr-Arg-His-Arg-Tyr-CONH2

[Lys18]PP (SEQ ID No:23).

H2N-Ala-Pro-Leu-Glu-Pro-Val-Tyr-Pro-Gly-Asp-Asn-Ala-Thr-Pro-Glu-Gln-Met-Lys-
Gln-Tyr-Ala-Ala-Asp-Leu-Arg-Arg-Tyr-Ile-Asn-Met-Leu-Thr-Arg-Pro-Arg-Tyr-CONH2

Ala-Arg-Arg-Arg-Ala-Ala-Arg-Ala-Ala-Arg-Arg-Arg-Ala-Ala-Arg-Ala-Ala-Arg-Arg-

Arg-Ala-Ala-Arg-Ala-PP (SEQ ID No:24).

H2N-Ala-Arg-Arg-Arg-Ala-Ala-Arg-Ala-Ala-Arg-Arg-Arg-Ala-Ala-Arg-Ala-Ala-Arg-Arg-
Arg-Ala-Ala-Arg-Ala-Ala-Pro-Leu-Glu-Pro-Val-Tyr-Pro-Gly-Asp-Asn-Ala-Thr-Pro-Glu-
Gln-Met-Ala-Gln-Tyr-Ala-Ala-Asp-Leu-Arg-Arg-Tyr-Ile-Asn-Met-Leu-Thr-Arg-Pro-Arg-
Tyr-CONH2

[Ala1,Glu4,Pro34]-PYY (SEQ ID No: 25)

H2N-Ala-Pro-Ile-Glu-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Leu-Val-Thr-Arg-Pro-Arg-Tyr-CONH2

S-Cys-Leu-Thr-Arg-Pro-Arg-Tyr-CONH2
|
S-Cys-Leu-Thr-Arg-His-Arg-Tyr-CONH2 (SEQ ID No: 26)

Lys-Lys-Lys-Lys-Lys-Lys-PP (SEQ ID No: 27)

H2N-Lys-Lys-Lys-Lys-Lys-Lys Ala-Pro-Leu-Glu-Pro-Val-Tyr-Pro-Gly-Asp-Asn-Ala-
Thr-Pro-Glu-Gln-Met-Ala-Gln-Tyr-Ala-Ala-Asp-Leu-Arg-Arg-Tyr-Ile-Asn-Met-Leu-Thr-
Arg-Pro-Arg-Tyr-CONH2

Lys-Lys-Lys-Lys-Lys-Lys-[His34]PP (SEQ ID No: 28)

H2N-Lys-Lys-Lys-Lys-Lys-Lys Ala-Pro-Leu-Glu-Pro-Val-Tyr-Pro-Gly-Asp-Asn-Ala-
Thr-Pro-Glu-Gln-Met-Ala-Gln-Tyr-Ala-Ala-Asp-Leu-Arg-Arg-Tyr-Ile-Asn-Met-Leu-Thr-
Arg-His-Arg-Tyr-CONH2

S-Cys-[N-(N'-hexadecanoyl)-gammaglutamoyl]-Lys-Thr-Arg-Leu-Arg-TyrCONH2
|
S-Cys-Leu-Thr-Arg-Leu-Arg-Tyr-CONH2 (SEQ ID. No: 29).

NHCOCH2CH2CH(COOH)NHCO(CH2)14CH3
|
S-Cys-Lys-Thr-Arg-Leu-Arg-TyrCONH2
|
S-Cys-Leu-Thr-Arg-Leu-Arg-Tyr-CONH2

N-Acetyl-PP (SEQ ID. No:30).
CH3CONH-Ala-Pro-Leu-Glu-Pro-Val-Tyr-Pro-Gly-Asp-Asn-Ala-Thr-Pro-Glu-Gln-Met-
Ala-Gln-Tyr-Ala-Ala-Asp-Leu-Arg-Arg-Tyr-Ile-Asn-Met-Leu-Thr-Arg-Pro-Arg-Tyr-
CONH2

N-(N'-hexadecanoyl)-gammaglutamoyl-PP (SEQ ID No: 31).
CH3(CH2)14CONHCH(COOH)CH2CH2CONH-Ala-Pro-Leu-Glu-Pro-Val-Tyr-Pro-Gly-
Asp-Asn-Ala-Thr-Pro-Glu-Gln-Met-Ala-Gln-Tyr-Ala-Ala-Asp-Leu-Arg-Arg-Tyr-Ile-Asn-
Met-Leu-Thr-Arg-Pro-Arg-Tyr-CONH2

[Nle17]PP (SEQ ID. No:32).
H2N-Ala-Pro-Leu-Glu-Pro-Val-Tyr-Pro-Gly-Asp-Asn-Ala-Thr-Pro-Glu-Gln-Nle-Ala-Gln-
Tyr-Ala-Ala-Asp-Leu-Arg-Arg-Tyr-Ile-Asn-Met-Leu-Thr-Arg-Pro-Arg-Tyr-CONH2

[Nle30]PP (SEQ ID. No:33).
H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Nle-Leu-Thr-Arg-Pro-Arg-Tyr-CONH2

[Nle17,Nle30]PP (SEQ ID. No:34).
H2N-Ala-Pro-Leu-Glu-Pro-Val-Tyr-Pro-Gly-Asp-Asn-Ala-Thr-Pro-Glu-Gln-Nle-Ala-Gln-
Tyr-Ala-Ala-Asp-Leu-Arg-Arg-Tyr-Ile-Asn-Nle-Leu-Thr-Arg-Pro-Arg-Tyr-CONH2

[N-(N'-hexadecanoyl)-gammaglutamoyl-Lys13,Nle30]PP (SEQ ID No: 35).
CH3(CH2)14CONHCH(COOH)CH2CH2CONH
|
H2N-Ala-Pro-Leu-Glu-Pro-Val-Tyr-Pro-Gly-Asp-Asn-Ala-Lys-Pro-Glu-Gln-Met-Ala-
Gln-Tyr-Ala-Ala-Asp-Leu-Arg-Arg-Tyr-Ile-Asn-Nle-Leu-Thr-Arg-Pro-Arg-Tyr-CONH2

[N-{(Ala-Arg-Arg-Arg-Ala-Ala-Arg-Ala)3}-Lys13]PP (SEQ ID No: 36)

Ala-Arg-Arg-Arg-Ala-Ala-Arg-Ala)3CONH
|
H2N-Ala-Pro-Leu-Glu-Pro-Val-Tyr-Pro-Gly-Asp-Asn-Ala-Lys-Pro-Glu-Gln-Met-Ala-
Gln-Tyr-Ala-Ala-Asp-Leu-Arg-Arg-Tyr-Ile-Asn-Met-Leu-Thr-Arg-Pro-Arg-Tyr-CONH2

[Nle17]PP2-36 (SEQ ID. No:37).
H2N-Pro-Leu-Glu-Pro-Val-Tyr-Pro-Gly-Asp-Asn-Ala-Thr-Pro-Glu-Gln-Nle-Ala-Gln-Tyr-
Ala-Ala-Asp-Leu-Arg-Arg-Tyr-Ile-Asn-Met-Leu-Thr-Arg-Pro-Arg-Tyr-CONH2

[Nle30]PP2-36 (SEQ ID. No:38).
H2N-Pro-Leu-Glu-Pro-Val-Tyr-Pro-Gly-Asp-Asn-Ala-Thr-Pro-Glu-Gln-Met-Ala-Gln-
Tyr-Ala-Ala-Asp-Leu-Arg-Arg-Tyr-Ile-Asn-Nle-Leu-Thr-Arg-Pro-Arg-Tyr-CONH2

[N-(N'-tetradecanoyl)-gammaglutamoyl-Lys13]PP2-36 (SEQ ID No: 39).
CH3(CH2)12CONHCH(COOH)CH2CH2CONH
|
H2N-Pro-Leu-Glu-Pro-Val-Tyr-Pro-Gly-Asp-Asn-Ala-Lys-Pro-Glu-Gln-Met-Ala-Gln-
Tyr-Ala-Ala-Asp-Leu-Arg-Arg-Tyr-Ile-Asn-Met-Leu-Thr-Arg-Pro-Arg-Tyr-CONH2

[N-{(Ala-Arg-Arg-Arg-Ala-Ala-Arg-Ala)3}-Lys13]PP2-36 (SEQ ID No: 40)
Ala-Arg-Arg-Arg-Ala-Ala-Arg-Ala)3CONH
|
H2N-Pro-Leu-Glu-Pro-Val-Tyr-Pro-Gly-Asp-Asn-Ala-Lys-Pro-Glu-Gln-Met-Ala-Gln-
Tyr-Ala-Ala-Asp-Leu-Arg-Arg-Tyr-Ile-Asn-Met-Leu-Thr-Arg-Pro-Arg-Tyr-CONH2

[Nle17,His34]-PP (SEQ ID. No:41).
H2N-Ala-Pro-Leu-Glu-Pro-Val-Tyr-Pro-Gly-Asp-Asn-Ala-Thr-Pro-Glu-Gln-Nle-Ala-Gln-
Tyr-Ala-Ala-Asp-Leu-Arg-Arg-Tyr-Ile-Asn-Met-Leu-Thr-Arg-His-Arg-Tyr-CONH2

[Nle30,His34]-PP (SEQ ID. No:42).
H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Nle-Leu-Thr-Arg-His-Arg-Tyr-CONH2

[Nle17,Nle30,His34]-PP (SEQ ID. No:43).
H2N-Ala-Pro-Leu-Glu-Pro-Val-Tyr-Pro-Gly-Asp-Asn-Ala-Thr-Pro-Glu-Gln-Nle-Ala-Gln-
Tyr-Ala-Ala-Asp-Leu-Arg-Arg-Tyr-Ile-Asn-Nle-Leu-Thr-Arg-His-Arg-Tyr-CONH2

[N-(N'-tetradecanoyl)-gammaglutamoyl-Lys13,His34]-PP (SEQ ID No: 44).
CH3(CH2)12CONHCH(COOH)CH2CH2CONH
|
H2N-Ala-Pro-Leu-Glu-Pro-Val-Tyr-Pro-Gly-Asp-Asn-Ala-Lys-Pro-Glu-Gln-Met-Ala-
Gln-Tyr-Ala-Ala-Asp-Leu-Arg-Arg-Tyr-Ile-Asn-Met-Leu-Thr-Arg-Pro-Arg-Tyr-CONH2

[N-{(Ala-Arg-Arg-Arg-Ala-Ala-Arg-Ala)3}-Lys13,His34]PP (SEQ ID No: 45)
(Ala-Arg-Arg-Arg-Ala-Ala-Arg-Ala)3CONH
|
H2N-Ala-Pro-Leu-Glu-Pro-Val-Tyr-Pro-Gly-Asp-Asn-Ala-Lys-Pro-Glu-Gln-Met-Ala-
Gln-Tyr-Ala-Ala-Asp-Leu-Arg-Arg-Tyr-Ile-Asn-Met-Leu-Thr-Arg-Pro-Arg-Tyr-CONH2

[Ala30,Pro34]-PYY (SEQ ID. No:46).
H2N-Tyr-Pro-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Ala-Val-Thr-Arg-Pro-Arg-Tyr-CONH2

[N-(N'-tetradecanoyl)-gammaglutamoyl-Lys13,Ala30,Pro34]-PYY (SEQ ID No:
47).
CH3(CH2)12CONHCH(COOH)CH2CH2CONH
|
H2N-Tyr-Pro-Ile-Lys-Pro-Glu-Ala-Pro-Gly-Glu-Asp-Ala-Lys-Pro-Glu-Glu-Leu-Asn-Arg-
Tyr-Tyr-Ala-Ser-Leu-Arg-His-Tyr-Leu-Asn-Ala-Val-Thr-Arg-Pro-Arg-Tyr-CONH2

[Cys2,N-(N'-tetradecanoyl)-gammaglutamoyl-Lys13,DCys27]-PP (SEQ ID No:
48).
S CH3(CH2)12CONHCH(COOH)CH2CH2CONH
| |
H2N-Ala-Cys-Leu-Glu-Pro-Val-Tyr-Pro-Gly-Asp-Asn-Ala-Lys-Pro-Glu-Gln-Met-Ala-
Gln-Tyr-Ala-Ala-Asp-Leu-Arg-Arg-(D-Cys)-Ile-Asn-Met-Leu-Thr-Arg-Pro-Arg-Tyr-
CONH2 |
S

S-Cys-Leu-Thr-Arg-Leu-Arg-Tyr-CONH2
|
S-Cys-Leu-Thr-Arg-Leu-Arg-Tyr-CONH2 (SEQ ID. No: 49).

S-Cys-Leu-Thr-Arg-Ile-Arg-Tyr-CONH2
|
S-Cys-Leu-Thr-Arg-Ile-Arg-Tyr-CONH2 (SEQ ID. No: 50).

[Leu34]-PP (SEQ ID. No:51).
H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Met-Leu-Thr-Arg-Leu-Arg-Tyr-CONH2

[Ile34]-PP (SEQ ID. No:52).
H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Met-Leu-Thr-Arg-Ile-Arg-Tyr-CONH2

[Phe34]-PP (SEQ ID. No:53).

H2N-Ala-Pro-Leu-Glu-Pro-Val-Tyr-Pro-Gly-Asp-Asn-Ala-Thr-Pro-Glu-Gln-Met-Ala-
Gln-Tyr-Ala-Ala-Asp-Leu-Arg-Arg-Tyr-Ile-Asn-Met-Leu-Thr-Arg-Phe-Arg-Tyr-CONH2

[Lys13jPP2-36 (SEQ ID No: 54)

H2N-Pro-Leu-Glu-Pro-Val-Tyr-Pro-Gly-Asp-Asn-Ala-Lys-Pro-Glu-Gln-Met-Ala-Gln-
Tyr-Ala-Ala-Asp-Leu-Arg-Arg-Tyr-Ile-Asn-Met-Leu-Thr-Arg-Pro-Arg-Tyr-CONH2

[N-(8-(8-gammaglutamoylamino-octanoylamino)-octanoyl)- [Lys13]PP2-36 (SEQ
ID No: 55)

H2NCH(COOH)CH2CH2CONH(CH2)7CONH(CH2)7CONH
|
H2N-Pro-Leu-Glu-Pro-Val-Tyr-Pro-Gly-Asp-Asn-Ala-Lys-Pro-Glu-Gln-Met-Ala-Gln-
Tyr-Ala-Ala-Asp-Leu-Arg-Arg-Tyr-Ile-Asn-Met-Leu-Thr-Arg-Pro-Arg-Tyr-CONH2

### APPENDIX

Our copending International Patent application entitled "Y2 Selective receptor agonists for Therapeutic Interventions" describes the use of Y2 selective agonists for various therapeutic and cosmetic treatments, for example for example for treatment of obesity and overweight, and conditions in which these are considered contributory factors.

A Y2 receptor agonist according to that application is one which
(a) is a PP-fold peptide or PP-fold peptide mimic selected from PYY, NPY, PYY mimics and NPY mimics which have a C-terminal Y2 receptor-recognition amino acid sequence and
   have no tyrosine residue corresponding to Tyr1 of NPY and/or
   have no proline residue corresponding to Pro2 of NPY and/or
   have no serine, asparagine, glutamine, threonine, leucine, isoleucine, valine, methionine, tryptophane, tyrosine or phenylalanine residue corresponding to Ser3 of NPY
   have no lysine or arginine residue corresponding to Lys4 of NPY and/or
   have a residue other than leucine in a position corresponding to Leu 24 in NPY and/or
   have a residue other than arginine in a position corresponding to Arg25 in NPY and/or
   have a residue other than histidine in a position corresponding to His26 in NPY and/or
   have a residue other than isoleucine in a position corresponding to Ile28 in NPY and/or
   have a residue other than asparagine in a position corresponding to Asn29 in NPY and/or
   have a residue other than leucine or methionine in a position corresponding to Leu30 in NPY; or
(b) is a PP-fold peptide or PP-fold peptide mimic selected from PP and PP-mimics which have a C-terminal Y2 receptor-recognition amino acid sequence and
   have no proline in a position corresponding to Pro2 in PP and/or
   have no isoleucine in a position corresponding to Ile3 in PP and/or
   have no glutamic acid in a position corresponding to Glu4 of PP; or
(c) (i) comprises a C-terminal Y2 receptor-recognition amino acid sequence fused at its N-terminus to an amphiphilic amino acid sequence domain comprising at least one alpha helical turn adjacent the N-terminus of the said Y2 receptor-recognition sequence, said turn being constrained in a helical configuration by a covalent intramolecular link, and (ii), in the case where the agonist has an N-terminal structure analogous to NPY or PYY, has one or more of the modifications listed in (a) above and, in the case where the agonist has an N-terminal structure analogous to PP, has one or more of the modifications listed in (b) above.

In general, type (a) agonists of that appliction are PP-fold analogues of NPY or PYY, or PP-fold mimics of NPY or PYY, which have modifications to reduce substantially their potencies towards Y1 receptors, including those which have a residue other than Tyr, Trp or Phe in a position corresponding to Tyr1 of NPY and/or a residue other than Pro in a position corresponding to Pro2 of NPY and/or a residue other than Ile, Leu, Val, Phe, Trp, Tyr, Ser, Thr, and Asn in a position corresponding to Ser3 of NPY and/or a residue other than lysine or arginine in a position corresponding to Lys4 of NPY. Such agonists include peptides having a sequence identical to that of human NPY except that there is a residue other than Tyr, Trp or Phe in position 1 and/or a residue other than Pro in position 2 and/or a residue other than Ile, Leu, Val, Phe, Trp, Tyr, Ser, Thr, and Asn in position 3 and/or a residue other than lysine or arginine in position 4.

In general, type (b) agonists of that application are PP-fold analogues of PP, or PP-fold mimics of PP, which have modifications to reduce substantially their potencies towards Y4 receptors, and to increase their potencies towards Y2 receptors. They include agonists having a Gln or a structurally similar residue in position 34 and a residue other than Pro, Tyr, Phe, and Trp in a position corresponding to Pro2 in PP and/or a residue other than Ile, Leu, Met, and Val in a position corresponding to Ile3 in PP and/or a residue other than Glu or Asp in a position corresponding to Glu4 of PP. One subset of agonists of this type consists of peptides having a sequence identical to that of human PP except that there is Gln residue in position 34 and a residue other than Pro, Tyr, Phe, and Trp in position 2 and/or a residue other than Ile, Leu, Met, and Val in position 3 and/or a residue other than Glu or Asp in position 4.

*Type* (c) agonists of that application are characterised by an intramolecular link, which either has no equivalent in the native NPY, PYY or PP peptides or which correspond to or substitute a non-covalent interaction with a covalent link, and includes those with a sequence identical to that of human PYY except that a helical turn-constraining intramolecular link extends from an amino acid residue in the amphiphilic domain to a linkage point in the N-terminal part of the polyproline domain. Such a link may be between residues in positions 5 and 20 or 8 and 16 or especially positions 2 and 27, for example a disulfide link between a D-Cys in position 27 and a Cys in position 2. In another set of agonists of type (c), the helical turn-constraining intramolecular link extends between residues in the last helical turn of the alpha helix domain, for example a lactam link formed between Lys and Glu residues in the helical turn, or between a residue in the C-terminal Y2 recognition amino acid sequence and a residue in the last helical turn of the alpha helix domain.

Being Y2 selective, all three types of agonist with which that application is concerned have a C-terminal Y2 receptor-recognition amino acid sequence. One set of C-terminal Y2 receptor-recognition amino acid sequences present in agonists of the invention is represented by -X-Thr-Arg-Gln-Arg-Tyr-C(=O)NR¹R² wherein X is a not a basic or acidic residue, R¹ and R¹ are independently hydrogen or C₁-C₆ alkyl, or a conservatively substituted variant thereof in which Thr is replaced by His or Asn and/or Tyr is replaced by Trp or Phe; and/or Arg is replaced by Lys. Boyt R¹ and R₂ may be hydrogen. One particular C-terminal Y2 receptor-recognition amino acid sequence is -Ile-Thr-Arg-Gln-Arg-Tyr-C(=O)NH₂, and another is -Ala-Thr-Arg-Gln-Arg-Tyr-C(=O)NH₂.

In all three types (a), (b) or (c), the C-terminal sequence comprising the Y2 receptor-recognition amino acid sequence may be fused at its N-terminus to an amphiphilic amino acid sequence domain comprising at least one alpha helical turn adjacent the N-terminus of the said epitope, and an N-terminal amino sequence of at least two amino acids, the said C- and N-terminal amino acid sequences being joined by peptide bonds to a linker radical, which may be a straight or branched chain alkylene radical, optionally containing one or more double or triple bonds. For example [Cys2, Aoc5-24,D-Cys27]PYY

Specific examples of Y2 selective agonists referred to in that application are:
PYY2-36
NPY2-36
[D-Ala1]PYY
[D-Ala2]PYY
[Ala28]PYY
[Ala30]PYY
[Ala31]PYY
[Lys4,Gln34]PP
[Cys2,D-Cys27]PYY
[Cys2,D-Cys27]NPY
[Cys2,Ile3,D-Cys27,Val31]NPY
[Cys2, Aoc5-24,D-Cys27]NPY
[Cys2, Ile3,Aoc5-24,D-Cys27,Val31]NPY
[Lys28,Glu32]PYY25-36
[Glu28,Lys32]PYY25-36
[D-Ala2]PYY2-36
[Lys4,Leu17,Gln34]PP
[Lys4,Leu17,Leu30,Gln34]PP
[Lys4,Nle17,Nle30,Gln34]PP
[Cys2,Ile3,D-Cys27,Leu28,Val31]NPY
[Cys2,Ile3,Nle17,D-Cys27,Nle28,Val31]NPY
[Cys2, Ile3,Aoc5-24,D-Cys27,Val31]NPY
[Cys2,Lys13,D-Cys27]PYY

## Claims

1. The use of a Y4 receptor agonist other than PP (SEQ ID No: 3), [Cys2,DCys27]-PP (SEQ ID. No:4) or PP2-36 (SEQ ID. No:10)., which is selective for the Y4 receptor over the Y1 and Y2 receptors, in the preparation of a composition for (i) reducing food intake, (ii) treatment of obesity or overweight, (iii) decreasing intestinal secretion or decreasing the rate of gastric emptying or (iv)treatment of a condition in which obesity or overweight is considered a contributory factor, selected from bulimia, bulimia nervosa, Syndrome X (metabolic syndrome), diabetes, type 2 diabetes mellitus or Non Insulin Dependent Diabetes Mellitus (NIDDM), hyperglycemia, insulin resistance, impaired glucose tolerance, cardiovascular disease, hypertension, atherosclerosis, coronary artery disease, myocardial infarction, peripheral vascular disease, stroke, thromboembolic disease, hypercholesterolemia, hyperlipidemia, gallbladder disease, osteoarthritis, sleep apnea, polycystic ovarian syndrome, and cancer of the breast, prostate, or colon,
(a) the said agonist being a PP-fold peptide or PP-fold peptide mimic which has
(i) a C-terminal Y4 receptor-recognition amino acid sequence represented by -X-Thr-Arg-X³-Arg-Tyr-C(=O)NR¹R² wherein R¹ and R¹ are independently hydrogen or C₁-C₆ alkyl X is Val, Ile, Leu or Ala, and X³ is a residue other than Gln, or a conservatively substituted variant thereof in which Thr is replaced by His or Asn and/or Tyr is replaced by Trp or Phe; and/or Arg is replaced by Lys, and
(ii) an N-terminal Y receptor-recognition amino acid sequence represented by H₂N-X¹-Pro-X²-(Glu or Asp)- wherein X¹ is not present or is any amino acid residue, and X² is Leu, Ile or Ser or a conservative substitution thereof, or
(b) the said agonist comprising
a C-terminal Y4 receptor-recognition sequence as defined in (i) above,
said sequence being fused to an amphiphilic amino acid sequence domain comprising at least one alpha helical turn adjacent the N-terminus of the said hexapeptide sequence,
said turn being constrained in a helical configuration by a covalent intramolecular link, and optionally
an N- terminal sequence which commences with a Y4 receptor-recognition amino acid sequence as defined in (ii) above; or
(c) the said agonist comprising two covalently linked C-terminal Y4 receptor-recognition amino acid sequences each of which comprises the last four residues of the sequence defined in (i) above.

2. The use as claimed in claim 1 wherein, in the C terminal Y4 receptor-recognition amino acid sequence of the agonist, R¹ and R² are each hydrogen.

3. The use as claimed in claim 1 or claim 2 wherein, in the C-terminal Y4 receptor-recognition sequence, residue X³ is not Asn, Lys, Arg, Asp or Glu.

4. The use as claimed in claim 1 or claim 2 wherein, in the C-terminal Y4 receptor-recognition sequence, residue X³ is Pro, His, or a non-natural Pro analogue selected from 4-hydroxyproline, azetidine-2-carboxylic acid, azetidine-3-carboxylic acid, azaproline, and 1-aminocyclobutanecarboxylic acid.

5. The use as claimed in any of the preceding claims wherein, in the C-terminal Y4 receptor-recognition amino acid sequence of the agonist, residue X is Leu.

6. The use as claimed in claim 1 wherein the agonist comprises a C-terminal heptapeptide represented by -X^{A}-X-Thr-Arg-X³-Arg-Tyr-C(=O)NR¹R² wherein the residue X^{A} is Leu or Met, and the sequence -X-Thr-Arg-X³-Arg-Tyr-C(=O)NR¹R² is as defined in any of claims 1 to 5.

7. The use as claimed in claim 1 wherein the agonist comprises a C-terminal undecapeptide represented by -X^{C}-Tyr-X^{B}-Asn-X^{A}-X-Thr-Arg-X³-Arg-Tyr-C(=O)NR¹R² wherein the sequence -X^{A}-X-Thr-Arg-X³-Arg-Tyr-C(=O)NR¹R² is as defined in claim 9, X^{C} is His, Asn, Gln, Arg or Lys and X^{B} is Ile, Leu or Val.

8. The use as claimed in claim 1 wherein the agonist comprises the C-terminal undecapeptide sequence -Arg-Tyr-Ile-Asn-(Leu or Met)-Leu-Thr-Arg-(Pro or His)-Arg-Tyr-C(=O)NH₂, or -His-Tyr-(Ile or Leu)-Asn-Leu-(Val/Ile)-Thr-Arg-(Pro or His)-Arg-Tyr-C(=O)NH₂.

9. The use as claimed in any of the preceding claims wherein, in the N-terminal Y4 receptor-recognition amino acid sequence of the agonist, when present, the residue X¹ is Ala, or is absent, and the residue X² is Leu, Ile, or Ser.

10. The use as claimed in any of claims 1 to 8 which has the N-terminal sequence H₂N-Ala-Pro-Leu-Glu-, or H₂N- Pro-Leu-Glu-.

11. The use as claimed in any of the preceding claims wherein the agonist is of type (b), wherein the covalent intramolecular link in the agonist is (A) a disulfide link formed between an L- or D-Cys residue in the alpha helix and a Cys residue located in the N-terminal part of the agonist corresponding to the polyproline domain of a PP-fold peptide which extends antiparallel to the amphiphilic domain, or (b) a lactam link formed between Lys and Glu residues in the said helical turn, or between a Lys or Glu residue in the said helical turn and a Glu or Lys residue in the C-terminal Y4 receptor recognition sequence.

12. The use as claimed in any of claims 1 to 10 wherein the agonist is of type (a) or (b) and has both a C-terminal hexapeptide Y4 receptor-recognition sequence as defined in claim 1 or any of claims 2 to 9 and an N-terminal Y4 receptor-recognition sequence as defined in claim 1, clai 9 or claim 10, the said C-terminal hexapeptide sequence being fused at its N-terminus to an amphiphilic amino acid sequence domain comprising at least one alpha helical turn adjacent the N-terminus of the hexapeptide sequence, the said C- and N-terminal amino acid sequences being joined by peptide bonds to the carboxyl and amino groups respectively of an amino acid of formula NH₂(CH₂)ₙCO₂H wherein n is from 2 to 12.

13. The use as claimed in claim 1 wherein the agonist is of type (c), and the two sequences are linked by a crosslink between one pair of residues located at least 4 residues from the C terminus of each of the two sequences, or are linked by a pair of crosslinks between two pairs of residues located at least 4 residues from the C terminus of each of the two sequences, and wherein the crosslink(s) is/are constituted by a disufide bridge between cysteine residues, or by an amide bond between the 3-amino group of a 2,3-propionic acid residue in one sequence and a carboxyl group of a side chain of a residue in the other sequence, or by a -(CH₂)₁₋₆-bridge formed by a bis-amino acid HOOCCH(NH₂)(CH₂)₁₋₆CH(NH₂)COOH, the ends of which form a residue in each of the respective sequences.

14. The use as claimed in claim 1 wherein the agonist is selected from
[Lys28,Glu32]PP25-36 (SEQ ID. No:5).
[Glu28,Lys32]PP25-36 (SEQ ID. No:6).
[Cys2,Aoc5-24,Dcys27]-PP (SEQ ID. No:9).
[His34]-PP (SEQ ID. No:11).
[Ala1,Pro34]-PYY (SEQ ID. No:12).
[Ala2,Pro34]-PYY (SEQ ID. No:13).
[Glu4,Pro34]-PYY (SEQ ID. No:14).
[Arg26,Pro34]-PYY (SEQ ID. No:15).
[Ile28,Pro34]-PYY (SEQ ID. No:16).
[Met30,Pro34]-PYY (SEQ ID. No:17).
[Ala1,Glu4,Pro34]-PYY (SEQ ID No: 25)
[Nle17]PP (SEQ ID. No:32).
[Nle30]PP (SEQ ID. No:33).
[NIe17,NIe30]PP (SEQ ID. No:34).
[NIe17]PP2-36 (SEQ ID. No:37).
[Nle30]PP2-36 (SEQ ID. No:38).
[NIe17,His34]-PP (SEQ ID. No:41).
[Nle30,His34]-PP (SEQ ID. No:42).
[NIe17,NIe30,His34]-PP (SEQ ID. No:43).
[Ala30,Pro34]-PYY (SEQ ID. No:46).
[Leu34]-PP (SEQ ID. No:51).
[Ile34]-PP (SEQ ID. No:52).
[Phe34]-PP (SEQ ID. No:53).
[Lys13]PP2-36 (SEQ ID No: 54) 
S-Cys-Thr-Arg-Pro-Arg-Tyr-CONH2
|
S-Cys-Thr-Arg-Pro-Arg-Tyr-CONH2 (SEQ ID. No:7).
 
S-Cys-Leu-Thr-Arg-Pro-Arg-Tyr-CONH2
|
S-Cys-Leu-Thr-Arg-Pro-Arg-Tyr-CONH2 (SEQ ID. No:18).
 
S-Cys-Leu-Thr-Arg-His-Arg-Tyr-CONH2
|
S-Cys-Leu-Thr-Arg-His-Arg-Tyr-CONH2 (SEQ ID. No:19).
 
S-Cys-Leu-Thr-Arg-Leu-Arg-Tyr-CONH2
|
S-Cys-Leu-Thr-Arg-Leu-Arg-Tyr-CONH2 (SEQ ID. No: 49).
 
S-Cys-Leu-Thr-Arg-Ile-Arg-Tyr-CONH2
|
S-Cys-Leu-Thr-Arg-Ile-Arg-Tyr-CONH2 (SEQ ID. No: 50).
and conservatively substituted analogues thereof.

15. The use as claimed in any of claims 1 to 12, or 14 wherein the agonist is acylated at its N-terminus to confer resistance to aminopeptidase activity.

16. The use as claimed in any of claims 1 to 15 wherein the agonist is as defined in any such claim, and comprises a serum albumin binding motif, or a glycosaminoglycan (GAG) binding motif, or a helix inducing motif, or is PEGylated.

17. A Y receptor agonist which is selective for the Y4 receptor over the Y1 and Y2 receptors selected from
[Lys28,Glu32]PP25-36 (SEQ ID. No:5).
[Glu28,Lys32]PP25-36 (SEQ ID. No:6).
[Cys2,Aoc5-24,Dcys27]-PP (SEQ ID. No:9).
[His34]-PP (SEQ ID. No:11).
[Alal,Pro34]-PYY (SEQ ID. No:12).
[Ala2,Pro34]-PYY (SEQ ID. No:13).
[Glu4,Pro34]-PYY (SEQ ID. No:14).
[Arg26,Pro34]-PYY (SEQ ID. No:15).
[Ile28,Pro34]-PYY (SEQ ID. No:16).
[Met30,Pro34]-PYY (SEQ ID. No:17).
[Ala1,Glu4,Pro34]-PYY (SEQ ID No: 25)
[Nle17]PP (SEQ ID. No:32).
[Nle30]PP (SEQ ID. No:33).
[Nle17,Nle30]PP (SEQ ID. No:34).
[Nle17]PP2-36 (SEQ ID. No:37).
[Nle30]PP2-36 (SEQ ID. No:38).
[Nle17,His34]-PP (SEQ ID. No:41).
[Nle30,His34]-PP (SEQ ID. No:42).
[Nle17,Nle30,His34]-PP (SEQ ID. No:43).
[Ala30,Pro34]-PYY (SEQ ID. No:46).
[Leu34]-PP (SEQ ID. No:51).
[Ile34]-PP (SEQ ID. No:52).
[Phe34]-PP (SEQ ID. No:53).
[Lys13]PP2-36 (SEQ ID No: 54) 
S-Cys-Thr-Arg-Pro-Arg-Tyr-CONH2
|
S-Cys-Thr-Arg-Pro-Arg-Tyr-CONH2 (SEQ ID. No:7).
 
S-Cys-Leu-Thr-Arg-Pro-Arg-Tyr-CONH2
|
S-Cys-Leu-Thr-Arg-Pro-Arg-Tyr-CONH2 (SEQ ID. No:18).
 
S-Cys-Leu-Thr-Arg-His-Arg-Tyr-CONH2
|
S-Cys-Leu-Thr-Arg-His-Arg-Tyr-CONH2 (SEQ ID. No:19).
 
S-Cys-Leu-Thr-Arg-Leu-Arg-Tyr-CONH2
|
S-Cys-Leu-Thr-Arg-Leu-Arg-Tyr-CONH2 (SEQ ID. No: 49).
 
S-Cys-Leu-Thr-Arg-Ile-Arg-Tyr-CONH2
|
S-Cys-Leu-Thr-Arg-Ile-Arg-Tyr-CONH2 (SEQ ID. No: 50).
N-Acetyl-PP (SEQ ID. No:30).
N-(N'-hexadecanoyl)-gammaglutamoyl-PP (SEQ ID No: 31).
[N-(N'-hexadecanoyl)-gammaglutamoyl-Lys13,Nle30]PP (SEQ ID No: 35).
[N-(N'-tetradecanoyl)-gammaglutamoyl-Lys13]PP2-36 (SEQ ID No: 39).
[N-(N'-tetradecanoyl)-gammaglutamoyl-Lys13,His34]-PP (SEQ ID No: 44).
[N-(N'-tetradecanoyl)-gammaglutamoyl-Lys13,Ala30,Pro34]-PYY (SEQ ID No: 47).
[Cys2,N-(N'-tetradecanoyl)-gammaglutamoyl-Lys13,DCys27]-PP (SEQ ID No: 48).
[N-(8-(8-gammaglutamoylamino-octanoylamino)-octanoyl)- [Lys13]PP2-36 (SEQ ID No: 55) 
S-Cys-[N-(N'-hexadecanoyl)-gammaglutamoyl]-Lys-Thr-Arg-Leu-Arg-Tyr-CONH2
|
S-Cys-Leu-Thr-Arg-Leu-Arg-Tyr-CONH2 (SEQ ID. No: 29).
[N-{(Ala-Arg-Arg-Arg-Ala-Ala-Arg-Ala)₃}-Lys13]PP (SEQ ID No: 36)
[N-{(Ala-Arg-Arg-Arg-Ala-Ala-Arg-Ala)₃}-Lys13]PP2-36 (SEQ ID No: 40)
[N-{(Ala-Arg-Arg-Arg-Ala-Ala-Arg-Ala)₃}-Lys13,His34]PP (SEQ ID No: 45)
and conservatively substituted analogues thereof.
